(19) Europäisches Patentamt
European Patent Office
Office européen des brevets

(11) Veröffentlichungsnummer: **0 011 599**
**B1**

(12) **EUROPÄISCHE PATENTSCHRIFT**

(45) Veröffentlichungstag der Patentschrift: **17.02.82**

(21) Anmeldenummer: **79810123.4**

(22) Anmeldetag: **12.10.79**

(51) Int. Cl.³: **C 07 C 87/14,** C 07 C 87/28, C 07 C 85/11, C 07 C 85/20, C 07 D 307/52, C 08 G 59/50 // C08G69/26

# (54) Substituierte 1,11-Diaminoundecane, Verfahren zu deren Herstellung und ihre Verwendung.

(30) Priorität: **18.10.78 CH 10770/78**

(43) Veröffentlichungstag der Anmeldung: **28.05.80 Patentblatt 80/11**

(45) Bekanntmachung des Hinweises auf die Patenterteilung: **17.02.82 Patentblatt 82/7**

(84) Benannte Vertragsstaaten: **CH DE FR GB IT NL**

(56) Entgegenhaltungen:
**DE-A-1 520 908**
**DE-A1-2 336 550**
**DE-A1-2 549 403**

(73) Patentinhaber: **CIBA-GEIGY AG, Patentabteilung Postfach, CH-4002 Basel (CH)**

(72) Erfinder: **Reinehr, Dieter, Dr., Wolfsheule 10, D-7842 Kandern (DE)**
Erfinder: **Pfeifer, Josef, Dr., Brunnmattstrasse 32, CH-4106 Therwil (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 599 B1

BUNDESDRUCKEREI BERLIN

## Substituierte 1,11-Diaminoundecane, Verfahren zu deren Herstellung und ihre Verwendung

Die vorliegende Erfindung betrifft neue substituierte 1,11-Diaminoundecane und Verfahren zu deren Herstellung.

Es ist bekannt, daß sich gegebenenfalls substituierte Alkylendiamine zur Herstellung von transparenten Polyamiden eignen. So werden z. B. in der deutschen Offenlegungsschrift 1 720 513 generisch kochbeständige, transparente Polyamide aus aromatischen Dicarbonsäuren und gegebenenfalls alkylsubstituierten Alkylendiaminen mit 1–10 C-Atomen in der Kette, die an mindestens einem der beiden endständigen C-Atome durch eine Alkylgruppe mit 1–4 C-Atomen substituiert sind, beschrieben. Die konkrete Offenbarung dieser Offenlegungsschrift beschränkt sich jedoch auf transparente Polyamide aus aromatischen Dicarbonsäuren und Alkylendiaminen der vorerwähnten Art mit höchstens 7 C-Atomen in der Kette. In den britischen Patentschriften 905 475 und 919 096 sind weitere transparente Polyamide aus Terephthalsäure, Isophthalsäure oder Gemischen davon und Hexamethylendiaminen mit mindestens drei durch Alkylsubstitution eingeführten C-Atomen in einer oder mehreren Seitenketten, wie 2,2,4- und 2,4,4-Trimethylhexamethylendiamin, 2-Methyl-4-äthylhexamethylendiamin und 2-Äthyl-4-methylhexamethylendiamin oder Isomerengemischen solcher Hexamethyendiamine beschrieben. Alkylendiamine eignen sich auch als Co-Kondensationskomponente für die Herstellung von transparenten Polyamiden aus 4,4'-Diaminodicyclohexylalkanen und aromatischen Dicarbonsäuren und gegebenenfalls weiteren Co-Kondensationskomponenten, wie Aminocarbonsäuren oder deren Lactame und aliphatische Dicarbonsäuren. Derartige Polyamide sind z. B. in der US-Patentschrift 3 597 400 und der deutschen Offenlegungsschrift 2 642 244 beschrieben. Diese vorbekannten Polyamide und Copolyamide lassen aber hinsichtlich der Wasseraufnahme, der Hydrolysebeständigkeit, der Wärmeformbeständigkeit und/oder der Dimensionsstabilität unter Feuchtigkeitseinwirkung zu wünschen übrig, wodurch auch die mechanischen und elektrischen Eigenschaften dieser Polyamide beeinträchtigt werden. Die genannten Polyamide sind ferner zum Teil nur schwer thermoplastisch verarbeitbar oder aber spröde Produkte.

Es ist ferner bekannt, daß aliphatische Diamine als Härter für Epoxidharze eingesetzt werden können. So werden z. B. in der DE-OS 2 549 403 1,10-substituierte 1,10-Diaminodecane zur Härtung von Epoxidharzen verwendet.

Es wurden nun neue Diamine gefunden, die sich zur Herstellung von transparenten Polyamiden eignen, die von den oben genannten Nachteilen frei sind. Die neuen Diamine sind ebenfalls wirksame Härter für Epoxidharze.

Die neuen substituierten 1,11-Diaminoundecane entsprechen der Formel I

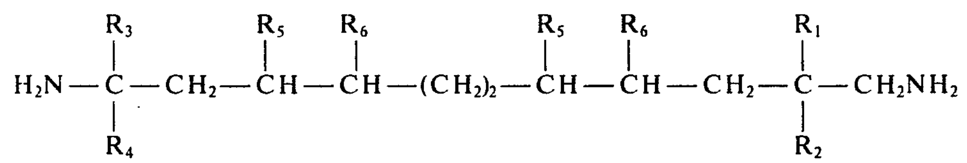

(I)

worin

$R_1$ Alkyl mit 1–12 C-Atomen,

$R_2$ Wasserstoff oder Alkyl mit 1–12 C-Atomen,

$R_3$ Alkyl mit 1–12 C-Atomen, Cycloalkyl mit 4–12 Ring-C-Atomen, Aralkyl mit 7 oder 8 C-Atomen, das mit einer oder mehreren $C_1-C_4$ Alkylgruppen substituiert sein kann, Pyridyl, Furyl oder Thienyl,

$R_4$ Wasserstoff, Alkyl mit 1–12 C-Atomen, Cycloalkyl mit 4–12 Ring-C-Atomen, Aralkyl mit 7 oder 8 C-Atomen oder gegebenenfalls mit einer oder mehreren $C_1-C_4$ Alkylgruppen substituiertes Aryl

oder $R_1$ und $R_2$ und/oder $R_3$ und $R_4$ zusammen Alkylen mit 3–11 C-Atomen und

$R_5$ und $R_6$ unabhängig voneinander Wasserstoff oder Methyl

bedeuten.

Durch $R_1$ bis $R_4$ dargestellte Alkylgruppen können geradkettig oder verzweigt sein. Alkylgruppen $R_1$, $R_2$ und $R_4$ weisen bevorzugt 1–5 C-Atome auf und sind geradkettig. Alkylgruppen $R_3$ weisen mit Vorteil 1–7 C-Atome auf; besonders bevorzugt sind verzweigte Alkylgruppen $R_3$ mit 3–7 C-Atomen. Beispiele von Alkylgruppen $R_1$ bis $R_4$ sind: Die Methyl-, Äthyl-, n-Propyl-, Isopropyl-, n-, sek- und tert-Butyl-, n-Pentyl-, 2- oder 3-Pentyl-, n-Hexyl-, 2- oder 3-Heptyl-, n-Octyl-, n-Decyl- und n-Dodecylgruppe.

Cycloalkylgruppen $R_3$ und $R_4$ können auch durch $C_{1-4}$-Alkylgruppen substituiert sein. Insbesondere handelt es sich dabei um durch eine Methyl- oder Äthylgruppe substituiertes Cycloalkyl. Bevorzugt sind Cycloalkylgruppen $R_3$ und $R_4$ jedoch unsubstituiert und weisen 5–8 Ring-C-Atome auf. Besonders bevorzugt sind die Cyclopentyl- und vor allem die Cyclohexylgruppe.

Als Aralkylgruppen $R_3$ und $R_4$ kommen vor allem die Benzyl-, Methylbenzyl- oder Phenyläthylgruppe

in Betracht. Stellt $R_3$ oder $R_4$ mit $C_1-C_4$ Alkylgruppen substituiertes Aryl dar, so kommen Alkylgruppen mit insbesondere 1 oder 2 C-Atomen in Betracht. Arylgruppen $R_3$ und $R_4$ sind bevorzugt nur durch eine Alkylgruppe substituiert. Besonders bevorzugt sind die 1- oder 2-Naphthylgruppe, durch eine Alkylgruppe mit 1–4 und besonders 1 oder 2 C-Atomen substituiertes Phenyl und ganz besonders unsubstituiertes Phenyl.

Als Pyridyl-, Furyl- oder Thienylgruppen $R_3$ kommen vor allem die Pyridyl-3-, Pyridyl-4-, Furyl-2- und Thienyl-2-gruppen in Betracht.

Durch $R_1$ und $R_2$ und/oder $R_3$ und $R_4$ dargestellte Alkylengruppen weisen bevorzugt 4–7 C-Atome auf. Insbesondere handelt es sich dabei um die Tetramethylen- und ganz besonders die Pentamethylengruppe.

Bevorzugt sind Verbindungen der Formel I, worin $R_1$ Alkyl mit 1–5 C-Atomen, $R_2$ Wasserstoff oder Alkyl mit 1–5 C-Atomen oder $R_1$ und $R_2$ zusammen Alkylen mit 4–7 C-Atomen, $R_3$ Alkyl mit 1–7 C-Atomen, Cycloalkyl mit 5–8 C-Atomen oder unsubstituiertes Phenyl, $R_4$ Wasserstoff oder Alkyl mit 1–5 C-Atomen und $R_5$ und $R_6$ je Wasserstoff bedeuten.

Besonders bevorzugt sind Verbindungen der Formel I, worin $R_1$ Alkyl mit 1–5 C-Atomen, $R_2$ Alkyl mit 1–5 C-Atomen oder Wasserstoff oder $R_1$ und $R_2$ zusammen Alkylen mit 4–7 C-Atomen, $R_3$ verzweigtes Alkyl mit 3–7 C-Atomen oder Cycloalkyl mit 5–8 C-Atomen, $R_4$ Wasserstoff und $R_5$ und $R_6$ je Wasserstoff bedeuten. Ganz besonders bevorzugt sind Verbindungen der Formel I, worin $R_1$ und $R_2$ je Methyl oder Äthyl oder zusammen $C_{4-7}$-Alkylen, besonders Pentamethylen, $R_3$ Isopropyl, 3-Pentyl, $C_{5-8}$-Cycloalkyl, besonders Cyclohexyl, und $R_4$, $R_5$ und $R_6$ je Wasserstoff bedeuten.

Spezielle, bevorzugte erfindungsgemäße Verbindungen der Formel I sind solche, die den Formeln IX bis XI entsprechen:

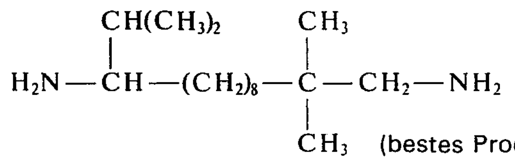
(bestes Produkt!)

(IX)

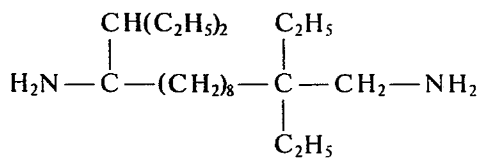

(X)

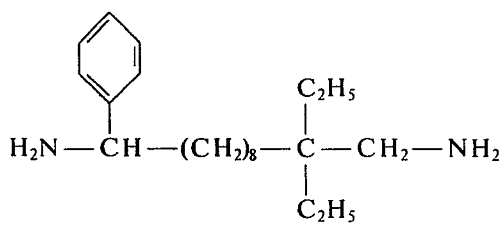

(XI)

Die Verbindungen der Formel I können dadurch hergestellt werden, daß man

A) eine Verbindung der Formel II

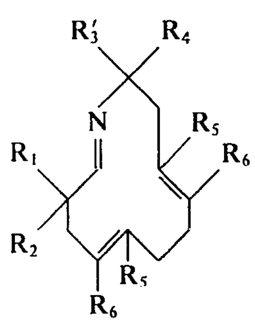

(II)

worin $R_1$, $R_2$ und $R_4$ bis $R_6$ die unter Formel I angegebene Bedeutung haben und $R'_3$ dasselbe wie

$R_3$ in Formel I oder, wenn $R_4$ Wasserstoff ist, auch

$$-CH{=}CH-\text{Alkyl oder} -C(\text{Alkyl}){=}CH-\text{Alkyl}$$

mit je 1–4 C-Atomen in den Alkylteilen darstellt, mit einer Verbindung der Formeln III a oder III b

$$H_2N-Y \qquad \text{(III a)}$$

oder

$$[H_2NY \cdot H^{\oplus}]_n \quad X^{\ominus n} \qquad \text{(III b)}$$

worin X das Anion einer anorganischen, unter den Reaktionsbedingungen nicht oxidierenden Säure, n eine der Wertigkeit von X entsprechende ganze Zahl und Y einer der Gruppen

$-OH$, $-NH_2$,

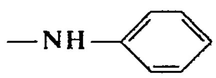

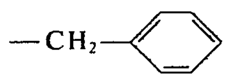

oder $-NHCONH_2$

darstellen, zu einer Verbindung der Formel IV a umsetzt

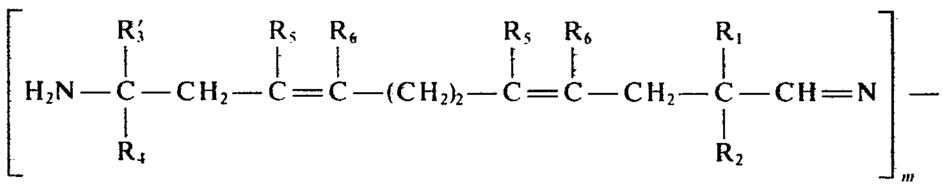

(IV a)

in der m die Zahl 1 oder 2 und Y' bei m = 1 die obige Bedeutung hat und bei m = 2 die direkte Bindung darstellt, oder

B) eine Verbindung der Formel II katalytisch zu einer Verbindung der Formel V

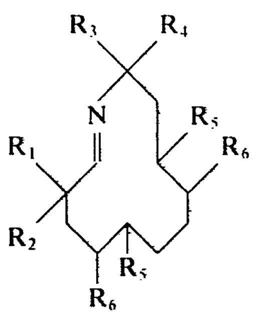

(V)

hydriert, wobei $R_1$ bis $R_6$ die unter Formel I angegebene Bedeutung haben, und die Verbindung der Formel V mit einer der Verbindungen der Formeln III a oder III b zu einer Verbindung der Formel IV b

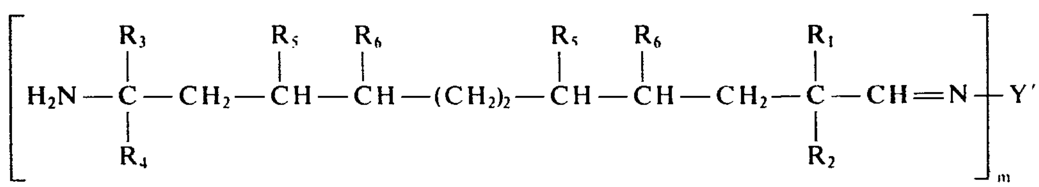

(IV b)

in der m und Y' die in Formel IVa angegebene Bedeutung haben, umsetzt und die Verbindung der Formel IVa oder IVb katalytisch zu einer Verbindung der Formel I hydriert.

Stellt $R_3'$ eine Gruppe $-CH=CH-$Methyl oder $-C(Alkyl)=CH-$Alkyl dar, so sind die Alkylteile in diesen Gruppen bevorzugt, geradkettig und bedeuten insbesondere Methyl oder Äthyl.

Zwischenprodukte der Formel IVb können auch durch katalytische Hydrierung von Verbindungen der Formel IVa erhalten werden. Bevorzugt ist jedoch die Herstellung von Verbindungen der Formel IVb nach der unter B) beschriebenen Methode.

Die Umsetzung der 1-Aza-1,5,9-cyclododecatriene der Formel II und der 1-Aza-cyclododecene der Formel V mit den Verbindungen der Formeln IIIa oder IIIb wird zweckmäßig in wäßrigem Medium bei Temperaturen zwischen etwa 20 und 100° C vorgenommen. X stellt z. B. das Anion der Chlor- oder Bromwasserstoffsäure, der Schwefel- oder Phosphorsäure dar. Bevorzugt verwendet man als Verbindung der Formel IIIb Hydroxylamin-hydrochlorid, Hydroxylaminsulfat oder Hydroxylamin-hydrogensulfat.

Die Verbindungen der Formel II und V werden zweckmäßig in im wesentlichen stöchiometrischer Menge, bezogen auf die Verbindungen der Formeln IIIa oder IIIb, eingesetzt. Mit Vorteil verwendet man jedoch einen leichten Überschuß an Verbindungen der Formeln IIIa oder IIIb.

Bei den obigen Umsetzungen können intermediär Verbindungen der Formel VIa oder VIb

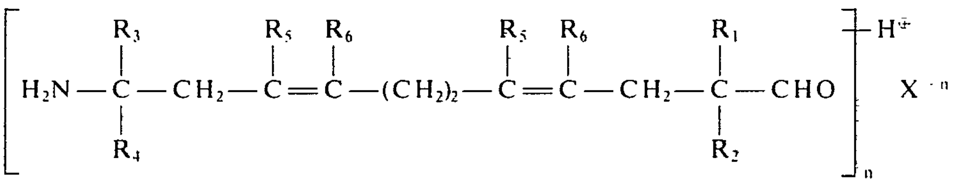

(VIa)

oder

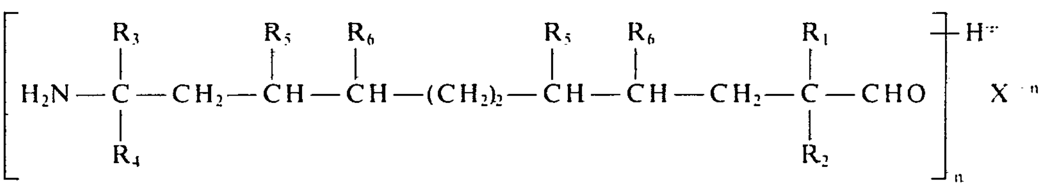

(VIb)

entstehen, wobei $R_1$ bis $R_6$, X und n die im Vorangehenden angegebene Bedeutung haben.

Im allgemeinen empfiehlt es sich, die Umsetzung unter Zusatz einer anorganischen, unter den Reaktionsbedingungen nicht-oxidierenden Säure, wie verdünnte HCl oder Schwefelsäure, vorzunehmen.

Nach beendeter Umsetzung zu den Verbindungen der Formel IVa oder IVb wird das Reaktionsgemisch zweckmäßig durch Zugabe einer geeigneten organischen oder anorganischen Base, wie Alkalimetall- oder Erdalkalimetallhydroxiden, -carbonaten oder -hydrogencarbonaten, tertiären Aminen, z. B. Triäthylamin oder Pyridin, neutralisiert. Bevorzugt verwendet man als Base ein Alkalimetallhydroxid, besonders Natrium- oder Kaliumhydroxid.

Die katalytische Hydrierung der 1-Aza-1,5,9-cyclododecatriene der Formel II zu den 1-Aza-cyclododecenen der Formel V, die katalytische Hydrierung von 11-Amino-undeca-4,8-dienal-verbindungen der Formel VIa zu 11-Amino-undecanalverbindungen der Formel VIb wie auch die Hydrierung der Verbindungen der Formeln IVa und IVb zu den Diaminen der Formel I können nach an sich bekannten Methoden, zweckmäßig in Gegenwart geeigneter inerter Lösungsmittel, vorgenommen werden. Die Hydrierungsreaktionen werden im allgemeinen in geschlossenem System bei einem Druck von etwa 1 bis 200 bar und insbesondere 1 bis 130 bar durchgeführt. Die Hydrierungstemperaturen liegen im allgemeinen zwischen etwa 0 und 150° C und insbesondere zwischen etwa 25 und 100° C.

Beispiele von geeigneten inerten organischen Lösungsmitteln für die Hydrierungen sind: Alkohole mit bis zu 6 C-Atomen, wie Methanol, Äthanol, Propanol, Isopropanol, Butanole und Pentanole; aliphatische und cycloaliphatische Kohlenwasserstoffe, wie n-Pentan, n-Hexan und Cyclohexan; cyclische Äther, wie Tetrahydrofuran, Tetrahydropyran und Dioxan; Äthylenglykol- und Diäthylenglykolmono- und -dialkyläther mit je 1–4 C-Atomen in den Alkylteilen, wie Äthylenglykol- und Diäthylenglykolmonomethyl und -monoäthyläther, Äthylenglykol- und Diäthylenglykoldimethyl- und -diäthyläther. Bevorzugte Lösungsmittel für die Hydrierung der 1-Aza-1,5,9-cyclododecatriene der Formel II und die Hydrierung von Verbindungen der Formel IVa zu Verbindungen der Formel IVb sind Cyclohexan und Tetrahydrofuran, während für die Hydrierung der Verbindungen der Formel IVa und IVb zu den Diaminen der Formel I Methanol als Lösungsmittel bevorzugt wird. Diese letztere Hydrierung wird gegebenenfalls in Gegenwart von flüssigem Ammoniak oder Natriumhydroxid vorgenommen.

Als Katalysatoren können an sich bekannte Hydrierungskatalysatoren eingesetzt werden, wie Platin-, Rhodium-, Palladium- und Ruthenium-Katalysatoren. Für die Hydrierung der 1-Aza-1,5,9-cyclododecatriene setzt man vorzugsweise Rhodium/Aluminiumoxid- oder Palladium/Kohle-Katalysatoren ein. Für die Hydrierung der Verbindungen der Formel IVa und IVb zu den Diaminen der Formel I werden Nickel-Katalysatoren, besonders Raney-Nickel, bevorzugt.

Verbindungen der Formel I, worin $R_3$ und/oder $R_4$ Cyclohexyl bedeuten, können auch durch katalytische Hydrierung der entsprechenden Verbindungen der Formel I mit $R_3$ und/oder $R_4$ = Phenyl hergestellt werden, wobei Katalysatoren der vorerwähnten Art, besonders Platin/Kohle-, Palladium/Kohle- oder Ruthenium/Kohle-Katalysatoren verwendet werden. Diese Hydrierungsreaktion wird zweckmäßig in Wasser, Alkoholen mit bis zu 6 C-Atomen oder Wasser/Alkohol-Gemischen durchgeführt.

Die Ausgangsprodukte der Formeln IIIa und IIIb sind bekannt. Die Verbindungen der Formel II können auf analoge Weise wie in Helv. Chim. Acta, 61, Fasc. 3, 1122–1124 (1978) beschrieben durch nickel-katalysierte Mischoligomerisation von 2-Aza-1,3-butadienen der Formel VII

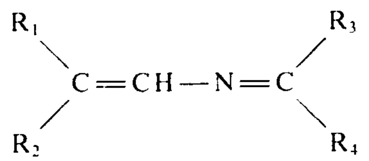
(VII)

mit Verbindungen der Formel VIII

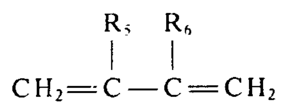
(VIII)

hergestellt werden, wobei $R_1$ bis $R_6$ die unter Formel I angegebene Bedeutung haben. Geeignete Katalysatorsysteme sind z. B. in der deutschen Offenlegungsschrift 2 330 087 beschrieben. Bevorzugt sind Katalysatoren, die in situ durch Reduktion einer kohlenoxidfreien Nickelverbindung, wie Nickelstearat und vor allem Nickelacetylacetonat, mit halogenfreien Metallarylen oder Metallalkylen, z. B. Äthoxy-diäthylaluminium, in Gegenwart eines Alkyl- oder Arylphosphins oder in Gegenwart eines Alkyl- oder Arylphosphits, erhalten werden.

Die obige Umsetzung wird zweckmäßig in Gegenwart eines inerten organischen Lösungsmittels, wie n-Hexan, n-Heptan, Benzol, Toluol, Diäthyläther oder Dioxan bei Temperaturen zwischen etwa −40° C und +150° C durchgeführt.

Die 2-Aza-1,3-butadiene der Formel VII sind größtenteils bekannt oder können beispielsweise wie folgt hergestellt werden:

– durch Umsetzung von Aldehyden $R_3-CHO$ oder Ketonen

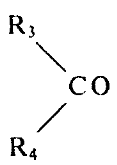

mit Alkenylaminen $H_2N-CH_2-C(R_1')=CH-R_2'$ [$R_1'$=H oder Alkyl mit 1–12 C-Atomen, $R_2'$ = H oder Alkyl C 1–11] und anschließende Isomerisierung der erhaltenen Verbindungen $R_2'-CH=C(R_1')-CH_2-N=C(R_3)(R_4)$ in Gegenwart von Katalysatoren, wie $K_2O/Al_2O_3$-Katalysatoren, Alkalimetall- oder Erdalkalimetallalkoholaten [vgl. z. B. B. A. Kazanskii et al., Zhurnal Organicheskoi Khimii, 6, No. 11, 2197–99 (1970); bzw. Akad. Nauk SSSR, Ser Khim., No. 9, 2038–2045 (1975) und Tetrahedron, 34, 833–839 (1978)];

– durch Umsetzung von Allylamin oder Methallylamin mit Aldehyden $(R_1)(R_2'')-CH-CHO$ [$R_2''$ wie $R_2$, aber ungleich H] und anschließende Isomerisierung der erhaltenen Verbindungen $(R_1)(R_2'')-CH-CH=N-CH_2-C(R)=CH_2$ [R = H oder Methyl] in Gegenwart von Katalysatoren, wie Kalium-tert-butylat;

– durch Umsetzung von Aldehyden $R_1-CH(R_2'')-CHO$ [$R_2''$ gleich wie $R_2$, aber ungleich H] mit Ammoniak (vgl. z. B. US-Patentschrift 2 319 848) und allfällige weitere Umsetzung der dabei erhaltenen Verbindungen $(R_1)(R_2'')-C=CH-N=CH-CH(R_2'')(R_1)$ mit geeigneten Ketonen oder Aldehyden (vgl. z. B. US-Patentschrift 3 706 802).

Nach Beendigung der Umsetzung und dem Entfernen des Katalysators und des Lösungsmittels können die Diamine der Formel I auf übliche Weise isoliert und gereinigt werden, beispielsweise mittels Destillation.

Grundsätzlich können die Diamine der Formel I auch aus den 1-Aza-1,5,9-cyclododecatrienen (oder -dodecenen) hergestellt werden, (Eintopfverfahren), indem man sie zuerst mit einer unter den Reaktionsbedingungen nicht oxidierenden Säure, (z. B. HCl, Schwefelsäure) behandelt und anschließend in Gegenwart von flüssigem Ammoniak katalytisch hydriert. Bei diesem Verfahren entstehen intermediär Verbindungen der Formeln IVa oder IVb mit Y = H und m = I.

Die Diamine der Formel I können beispielsweise zur Herstellung von Polykondensationsprodukten, vor allem Polyamiden, verwendet werden. Transparente Polyamide, die durch Polykondensation von Diaminen der Formel I, worin $R_3 \neq$ heterocyclischer Rest, mit aromatischen Dicarbonsäuren, vor allem Terephthalsäure und/oder Isophthalsäure und gegebenenfalls weiteren Diaminen und aliphatischen Dicarbonsäuren erhalten werden, zeichnen sich durch hohe Glasumwandlungstemperaturen und dementsprechend hohe Wärmeformbeständigkeit, gute thermoplastische Verarbeitbarkeit, z. B. nach dem Spritzguß- oder Extrusionsverfahren, geringe Wasseraufnahme verbunden mit verminderter Abhängigkeit der mechanischen und elektrischen Eigenschaften von der Umgebungsfeuchtigkeit und verbesserte Hydrolysebeständigkeit sowie Kochwasserbeständigkeit aus.

Weiterer Gegenstand der Erfindung ist auch die Verwendung der erfindungsgemäßen Diamine als Härter für Epoxidharze. Die entsprechenden erfindungsgemäßen härtbaren Gemische enthalten

a) ein Diamin der Formel I und
b) eine Polyepoxidverbindung (X) mit durchschnittlich mehr als einer Epoxidgruppe im Molekül,

wobei in den Gemischen auf 1 Äquivalent Epoxidgruppen der Polyepoxidverbindung (X) 0,5 bis 1,5 Äquivalente an Stickstoff gebundene, aktive Wasserstoffatome des jeweiligen Diamins der Formel I kommen.

Die als Härter verwendbaren Diamine können auch in Form eines Addukthärters (E) mit einer Aminzahl von 3 bis 7 Val/kg aus dem Diamin der Formel I und einer flüssigen Epoxidverbindung (Z) mit durchschnittlich mehr als einer Epoxidgruppe im Molekül eingesetzt werden. Gegebenenfalls wird Phenylglycid zugesetzt. In den Gemischen kommen auf 1 Äquivalent Epoxidgruppen der Polyepoxidverbindung (X) 0,8 bis 1,2 Äquivalente an die Stickstoffatome des Addukthärters (E) gebundene, aktive Wasserstoffatome.

Grundsätzlich kann auch nur ein Epoxidharz eingesetzt werden, d. h. Grundharz (X) und das in dem Addukthärter (E) enthaltene Epoxidharz (Z) sind die gleiche Verbindung.

Die erfindungsgemäßen härtbaren Gemische weisen die Vorteile auf, daß ihre Gebrauchsdauer groß ist, und daß sie zu Produkten hoher Flexibilität führen. Außerdem führen sie als Klebstoffe und Lacke zu einer ausgezeichneten Haftfähigkeit.

In den folgenden Beispielen wird die Erfindung näher erläutert. Darin bedeuten, wenn nicht ausdrücklich anders angegeben, Teile Gewichtsteile.

## Beispiel 1

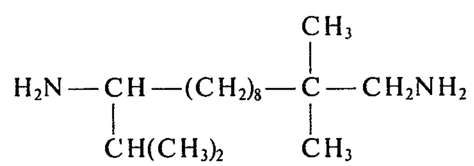

a) Unter Schutzgas (Argon) werden 2,57 g (0,01 Mol) Nickelacetylacetonat und 1,66 g (0,01 Mol) Triäthylphosphit in 120 g absolutem Toluol gelöst, worauf man die Lösung bei 20–25°C mit 1,3-Butadien sättigt. Anschließend werden unter schwachem Einleiten von 1,3-Butadien langsam 3,9 g (0,03 Mol) Äthoxydiäthylaluminium zugetropft. Es wird auf 60°C aufgeheizt, und unter starkem Einleiten von 1,3-Butadien werden innerhalb von 45 Minuten 122,5 g (0,98 Mol) N-Isobutyliden-2-methylpropenylamin [hergestellt durch Umsetzung von Isobutyraldehyd mit Ammoniak gemäß J. Org. Chem. 26, 1822–25 (1961)] so zugetropft, daß man eingeleitete Butadien gerade verbraucht wird. Nach beendetem Zutropfen wird noch 1 Stunde unter stetigem Einleiten von 1,3-Butadien bei 60°C nachgerührt und dann auf 20–25°C abgekühlt. Der Katalysator wird durch Zugabe von 0,32 g (0,01 Mol) Schwefel inaktiviert, und die Reaktionslösung wird destilliert. Durch anschließende Feindestillation erhält man 212,5 g (0,912 Mol) 3,3-Dimethyl-12-isopropyl-1-aza-1,5,9-cyclododecatrien; Sdp. 54–55°C/1,33 Pa; $n_D^{20}$ 1,4832.

b) 233,4 g (1 Mol) 3,3-Dimethyl-12-isopropyl-1-aza-1,5,9-cyclododecatrien werden innerhalb von 1 Stunde so zu 100 g 37%iger Salzsäure und 200 ml Wasser getropft, daß die Temperatur nicht über 80°C ansteigt. Dann wird auf 20–25°C abgekühlt und mit 69,5 g (1,0 Mol) Hydroxylaminhydrochlorid versetzt. Während einer Stunde werden unter Kühlung mit einem Wasserbad ca. 92 g (2,3 Mol) festes Natriumhydroxid zugegeben, bis der pH-Wert der wäßrigen Lösung 10–11 beträgt. Die sich abscheidende organische Phase wird abgetrennt und mit Wasser salzfrei gewaschen. Nach der Destillation erhält man 245 g (0,92 Mol) 2,2-Dimethyl-11-isopropyl-11-amino-undeca-4,8-dienal-oxim;

Sdp. 158 – 162° C/4 Pa; $n_D^{20}$ = 1,4930.

c) 490 g (1,84 Mol) 2,2-Dimethyl-11-isopropyl-11-aminoundeca-4,8-dienal-oxim werden in 2,4 Liter Methanol gelöst und zusammen mit ca. 200 g flüssigem Ammoniak unter Zugabe von 150 g Raney-Nickel in einem 6,3-Liter Stahlautoklaven eingefüllt. Es wird anschließend Wasserstoff bis zu einem Druck von 100 bar aufgepreßt und unter Rühren auf 100° C aufgeheizt. Man hydriert ca. 5 Stunden unter diesen Bedingungen, kühlt ab und läßt dann den Ammoniak und überschüssigen Wasserstoff abblasen. Bei der anschließenden Destillation im Hochvakuum erhält man 436 g (1,705 Mol) 1-Isopropyl-10,10-dimethyl-1,11-diaminoundecan als farblose, wasserklare Flüssigkeit; Ausbeute 92,6% d. Th.; Sdp. 87° C/4 Pa; $n_D^{20}$ = 1,4619.

Analyse für $C_{16}H_{36}N_2$ (Molgewicht 256,48):

| | | | |
|---|---|---|---|
| berechnet | C 74,93 | H 14,15 | N 10,92% |
| gefunden | C 74,88 | H 14,17 | N 10,79% |

MS-Spektrum:
Molekül-Peak 256, Bruchstückmassen 241, 227, 213, 196, 184, 128, 72, 56.

H-NMR-Spektrum $\tau$ (ppm):
7,45 (m) und 7,52 (s), 8,2 – 8,7 (m), 8,92 (s) und 9,03 (dd) und 9,08 (s) im Verhältnis 3 : 17 : 16.

## Beispiel 2

a) 466,8 g (2 Mol) 3,3-Dimethyl-12-isopropyl-1-aza-1,5,9-cyclododecatrien werden in 4 Liter Cyclohexan gelöst und bei 20 – 25° C und einem Anfangsdruck von 100 bar in Gegenwart von 50 g Rhodium/Aluminiumoxid während 4 Stunden in einem Stahlautoklaven hydriert. Nach dem Abdestillieren des Lösungsmittels erhält man als Hauptfraktion 425 g (1,79 Mol) 3,3-Dimethyl-12-isopropyl-1-aza-cyclododecen; Sdp. 92 – 94° C/4 Pa; $n_D^{20}$ = 1,4706.

b) Es wird wie in Beispiel 1b) beschrieben vorgegangen, jedoch unter Verwendung von 118,7 g (0,5 Mol) 3,3-Dimethyl-12-isopropyl-1-aza-cyclododecen, 35 g (0,5 Mol) Hydroxylaminhydrochlorid, 55 g 37%iger Salzsäure, 200 ml Wasser und 50 g (1,25 Mol) festen Natriumhydroxid. Nach der Destillation erhält man 127 g (0,47 Mol) 2,2-Dimethyl-11-isopropyl-11-amino-undecanal-oxim; Sdp. 145° C/4 Pa; $n_D^{20}$ = 1,4761.

c) Es wird wie in Beispiel 1c) beschrieben vorgegangen, jedoch unter Verwendung von 221 g (0,819 Mol) 2,2-Dimethyl-11-isopropyl-11-amino-undecanal-oxim. Nach der Destillation erhält man 202 g (0,789 Mol) 1-Isopropyl-10,10-dimethyl-1,11-diaminoundecan; Ausbeute 96,2% d. Th.

## Beispiel 3

a) Analog der in Beispiel 1a) beschriebenen Arbeitsweise werden 48,5 g (0,437 Mol) N-Propyliden-(2-methylpropenylamin) [1-Äthyl-4,4-dimethyl-2-aza-1,3-butadien] mit 61,0 g (1,13 Mol) 1,3-Butadien umgesetzt. Durch Destillation erhält man 62,0 g (0,283 Mol) 3,3-Dimethyl-12-äthyl-1-aza-1,5,9-cyclododecatrien; Sdp. 65 – 66° C/0,7 Pa; $n_D^{20}$ = 1,4864.

Das N-Propyliden-(2-methylpropenylamin) wurde wie folgt hergestellt: 25 g (0,223 Mol) Kalium-tert-butylat werden in einem Liter wasserfreiem Diäthyläther aufgeschlämmt. Dann tropft man innerhalb von 1 Stunde unter stetigem Rühren 921 g (8,3 Mol) Isobutyliden-allylamin so zu, daß die Temperatur des Reaktionsgemisches nicht über 20° C ansteigt. Nach beendetem Eintropfen wird noch 5 Stunden bei 20 – 22° C weitergerührt. Dann wird die Umsetzung unterbrochen, und das Lösungsmittel wird bei einer Badtemperatur von 40° C/27000 – 35000 Pa überdestilliert. Der Rückstand wird bei einer Badtemperatur von 70° C/13 Pa in eine mit $CO_2$/Methanol gekühlte Vorlage destilliert. Nach anschließender Feindestillation erhält man 808 g (7,93 Mol) N-Propyliden-(2-methylpropenylamin); Sdp. 122° C; $n_D^{20}$ = 1,471.

b) Es wird wie in Beispiel 1b) beschrieben vorgegangen, jedoch unter Verwendung von 219 g (1 Mol) 3,3-Dimethyl-12-äthyl-1-aza-1,5,9-cyclododecatrien, 147 g (1,5 Mol) Schwefelsäure und 82 g (0,5 Mol) Hydroxylaminsulfat.

Nach der Aufarbeitung erhält man 222 g (0,879 Mol) 2,2-Dimethyl-11-äthyl-11-amino-undeca-4,8-dienal-oxim als hochviskose Flüssigkeit; Sdp. 162 – 164° C/7 Pa.

c) Es wird wie in Beispiel 1c) beschrieben vorgegangen, jedoch unter Verwendung von 172,5 g (0,684 Mol) 2,2-Dimethyl-11-äthyl-11-amino-undeca-4,8-dienal-oxim. Nach der Destillation erhält man 160 g (0,661 Mol) 1-Äthyl-10,10-dimethyl-1,11-diaminoundecan; Ausbeute 96,5% d. Th.; Sdp. 93° C/7 Pa; $n_D^{20}$ = 1,4622.

Analyse für $C_{15}H_{34}N_2$ (Molgewicht 242,45):

| | | | |
|---|---|---|---|
| berechnet | C 74,31 | H 14,14 | N 11,56% |
| gefunden | C 74,35 | H 14,32 | N 11,71% |

MS-Spektrum:
Molekül-Peak 242, Bruchstückmassen 227, 213, 196, 184, 128, 98, 56.

$^1$H-NMR-Spektrum $\tau$ (ppm):
7,42 (m) und 7,58 (s), 8,69 (m), 8,92 (s), 9,08 (t) und 9,15 (s) im Verhältnis 3 : 18 : 4 : 9.

## Beispiel 4

a) Es wird wie in Beispiel 2a) beschrieben vorgegangen, jedoch unter Verwendung von 438 g (2 Mol) 3,3-Dimethyl-12-äthyl-1-aza-1,5,9-cyclodecatrien. Nach der Destillation erhält man 412 g (1,85 Mol) 3,3-Dimethyl-12-äthyl-1-aza-cyclododecen; Sdp. 61 – 63° C/4 Pa; n = 1,4721.

b) Es wird wie in Beispiel 1b) beschrieben vorgegangen, jedoch unter Verwendung von 223 g (1 Mol) 3,3-Dimethyl-12-äthyl-1-aza-cyclododecen, 82 g (0,5 Mol) Hydroxylaminsulfat, 150 g Schwefelsäure und 400 ml Wasser. Nach der Destillation erhält man 245 g (0,955 Mol) 2,2-Dimethyl-11-äthyl-11-amino-undecanal-oxim; Sdp. 155 – 160° C/7 Pa.

c) Es wird wie in Beispiel 1c) beschrieben vorgegangen, jedoch unter Verwendung von 275 g (1,075 Mol) 2,2-Dimethyl-11-äthyl-11-amino-undecanal-oxim. Nach der Destillation erhält man 215 g (0,887 Mol) 1-Äthyl-10,10-dimethyl-1,11-diaminoundecan; Ausbeute 82,6% d. Th.

## Beispiel 5

a) Auf analoge Weise wie in Beispiel 1a) beschrieben werden 495 g (3,41 Mol) N-Benzyliden-propenylamin mit 1,3-Butadien umgesetzt. Nach der Destillation erhält man 750 g (2,97 Mol) 3-Methyl-12-phenyl-1-aza-1,5,9-cyclododecatrien als cis-trans-Isomerengemisch (cis : trans = 65 : 35); Sdp. 112 – 113° C/1 Pa; n = 1,5505; Smp. (cis-Isomeres) = 57 – 58° C.

b) 502 g (1,98 Mol) 3-Methyl-12-phenyl-1-aza-1,5,9-cyclododecatrien werden innerhalb von 1,5 Stunden so zu 220 g 37%iger Salzsäure zugetropft, daß die Temperatur nicht über 80° C ansteigt. Dann wird auf Raumtemperatur (20 – 25° C) abgekühlt und mit 140 g (2,02 Mol) Hydroxylaminhydrochlorid versetzt. Während einer Stunde werden unter Kühlung mit einem Wasserbad ca. 185 g (4,6 Mol) festes Natriumhydroxid zugegeben, bis der pH der wäßrigen Lösung 10 – 11 beträgt. Die sich abscheidende organische Phase wird abgetrennt und mit Wasser neutral gewaschen. Man erhält 567 g (1,98 Mol) 2-Methyl-11-phenyl-11-amino-undeca-4,8-dienaloxim als hochviskose Flüssigkeit. MS-Spektrum: Molekül-Peak 286, Bruchstückmassen 269, 254, 214, 148, 121, 106.

c) Es wird wie in Beispiel 1c) beschrieben vorgegangen, jedoch unter Verwendung von 567 g (1,98 Mol) 2-Methyl-11-phenyl-11-amino-undeca-4,8-dienal-oxim. Nach der Destillation erhält man 441 g (1,6 Mol) 1-Phenyl-10-methyl-1,11-diaminoundecan; Ausbeute 80,7% d. Th.; Sdp. 138 – 140° C/1 Pa; n = 1,5095.

Analyse für $C_{18}H_{32}N_2$ (Molgewicht 276,47):

| | | | |
|---|---|---|---|
| berechnet | C 78,20 | H 11,67 | N 10,13% |
| gefunden | C 78,34 | H 11,83 | N 10,03% |

MS-Spektrum:
Molekül-Peak 276, Bruchstückmassen 230, 106, 79, 44.

$^1$H-NMR-Spektrum $\tau$ (ppm):
2,72 (s), 6,14 (t), 7,45 (m), 8,2 – 8,8 (m), 9,08 (d) im Verhältnis 5 : 1 : 2 : 21 : 3.

## Beispiel 6

a) 253 g (1 Mol) 3-Methyl-12-phenyl-1-aza-1,5,9-cyclododecatrien werden in 2 Liter Cyclohexan gelöst und bei 20 – 25° C und einem Anfangsdruck von 100 bar in Gegenwart von 40 g Rhodium/Aluminiumoxid während 4 Stunden in einem Stahlautoklaven hydriert. Nach dem Abdestillieren des Lösungsmittels erhält man als Hauptfraktion 242 g (0,94 Mol) 3-Methyl-12-phenyl-1-aza-cyclododecen als cis-trans-Isomerengemisch; Sdp. 112° C/4 Pa.

b) Es wird wie in Beispiel 1b) beschrieben vorgegangen, jedoch unter Verwendung von 146 g (0,568 Mol) 3-Methyl-12-phenyl-1-aza-cyclododecen, 47 g (0,286 Mol) Hydroxylaminsulfat, 60 g (0,61 Mol)

Schwefelsäure und 200 ml Wasser. Nach der Aufarbeitung wie in Beispiel 1b) beschrieben, erhält man 165 g (0,567 Mol) 2-Methyl-11-phenyl-11-amino-undecanal-oxim als hochviskose Flüssigkeit.

c) Es wird wie in Beispiel 1c) beschrieben vorgegangen, jedoch unter Verwendung von 165 g (0,567 Mol) 2-Methyl-11-phenyl-11-amino-undecanal-oxim. Nach der Destillation erhält man 110 g (0,399 Mol) 1-Phenyl-10-methyl-1,11-diaminoundecan; Ausbeute 70,3% d. Th.

Beispiel 7

a) Analog der in Beispiel 1a) beschriebenen Arbeitsweise werden 110 g (1,13 Mol) N-Isopropyliden-propenylamin (hergestellt durch Umsetzung von Aceton mit Allylamin und anschließender Isomerisierung; Zhurnal Organicheskoi Khimii, 6, Nr. 11, 2197-9 [1970]) mit 108 g (2 Mol) 1,3-Butadien umgesetzt. Nach der Destillation erhält man 187,0 g (0,91 Mol) 3,12,12-Trimethyl-1-aza-1,5,9-cyclododecatrien; Sdp. 55° C/4 Pa; $n_D^{20}$ = 1,4895.

b) Es wird wie in Beispiel 1b) beschrieben vorgegangen, jedoch unter Verwendung von 205 g (1 Mol) 3,12,12-Trimethyl-1-aza-1,5,9-cyclododecatrien, 82 g (0,5 Mol) Hydroxylaminsulfat, 110 g Schwefelsäure und 300 ml Wasser. Nach der Aufarbeitung und anschließender Destillation erhält man 205 g (0,86 Mol) 2,11,11-Trimethyl-11-aminoundeca-4,8-dienal-oxim: Sdp. 155° C/20 Pa; $n_D^{20}$ = 1,4950.

c) Es wird wie in Beispiel 1c) beschrieben vorgegangen, jedoch unter Verwendung von 202 g (0,848 Mol) 2,11,11-Trimethyl-11-amino-undeca-4,8-dienal-oxim. Nach der Destillation erhält man 173 g (0,758 Mol) 1,1,10-Trimethyl-1,11-diaminoundecan; Ausbeute 89,4% d. Th.; Sdp. 87° C/4 Pa; $n_D^{20}$ = 1,4585.

Analyse für $C_{14}H_{32}N_2$ (Molgewicht 228,42):

| | | | |
|---|---|---|---|
| berechnet | C 73,62 | H 14,12 | N 12,27% |
| gefunden | C 73,42 | H 14,20 | N 12,31% |

MS-Spektrum:
Molekül-Peak 228, Bruchstückmassen 213, 199, 182, 154, 126, 84, 70, 58.

$^1$H-NMR-Spektrum $\tau$ (ppm):
7,48 (m), 8,68 (s), 8,90 (s), 9,09 (d) im Verhältnis 2 : 21 : 6 : 3.

Beispiel 8

a) Analog der in Beispiel 1a) beschriebenen Arbeitsweise werden 273 g (2,02 Mol) N-2-Furyliden-propenylamin (hergestellt durch Umsetzung von 2-Furylaldehyd mit Allylamin und anschließende Isomerisierung in Gegenwart von Kalium-tert-butylat; Sdp. 60–62° C/67 Pa; $n_D^{20}$ = 1,6004) mit 1,3-Butadien umgesetzt. Nach der Destillation erhält man 210 g (0,865 Mol) 3-Methyl-12-(2-furyl)-1-aza-1,5,9-cyclododecatrien; Sdp. 106–108° C/4 Pa; $n_D^{20}$ = 1,5260.

b) Es wird wie in Beispiel 1b) beschrieben vorgegangen, jedoch unter Verwendung von 190 g (0,78 Mol) 3-Methyl-12-(2-furyl)-1-aza-1,5,9-cyclododecatrien, 54 g (0,78 Mol) Hydroxylaminhydrochlorid, 84 g Schwefelsäure, 300 ml Wasser und 65 g festem Natriumhydroxid. Die sich abscheidende organische Phase enthält 201 g (0,728 Mol) 2-Methyl-11-(2-furyl)-11-amino-undeca-4,8-dienal-oxim; $n_D^{20}$ = 1,5305.

c) Es wird wie in Beispiel 1c) beschreiben vorgegangen, jedoch unter Verwendung von 276 g (1 Mol) 2-Methyl-11-(2-furyl)-11-amino-undeca-4,8-dienal-oxim. Nach der Destillation erhält man 224 g (0,842 Mol) 1-(2-Furyl)-10-methyl-1,11-diaminoundecan; Ausbeute 84,2% d. Th.; Sdp. 135–138° C/7 Pa; $n_D^{20}$ = 1,4869.

Analyse für $C_{16}H_{30}N_2O$ (Molgewicht 266,43):

| | | | | |
|---|---|---|---|---|
| berechnet | C 72,13 | H 11,35 | N 10,52 | O 6,01% |
| gefunden | C 72,08 | H 11,38 | N 10,43 | O 6,25% |

MS-Spektrum:
Molekül-Peak 266, Bruchstückmassen 244, 220, 199, 96.

$^1$H-NMR-Spektrum $\tau$ (ppm):
2,69 (d), 3,72 (dd), 3,93 (d), 6,13 (t), 7,46 (m), 8,1–8,8 (m), 9,08 (d) im Verhältnis 1 : 1 : 1 : 1 : 2 : 21 : 3.

Beispiel 9

a) Es wird wie in den vorangehenden Beispielen beschrieben vorgegangen, jedoch unter Verwendung von 72,4 g (0,4 Mol) 1-(3-Pentyl)-4,4-diäthyl-2-aza-1,3-butadien (hergestellt durch

Umsetzung von 2-Äthylbutyraldehyd mit Ammoniak gemäß US-Patentschrift 2 319 948) und 48,4 g (0,895 Mol) 1,3-Butadien. Nach der Aufarbeitung erhält man 56,8 g (0,197 Mol) 3,3-Diäthyl-12-(3-pentyl)-1-aza-1,5,9-cyclododecatrien; Sdp. 90–92° C/0,13 Pa; $n_D^{20}$ = 1,4840.

b) Es wird wie in Beispiel 2a) beschrieben vorgegangen, jedoch unter Verwendung von 289 g (1 Mol) 3,3-Diäthyl-12-(3-pentyl)-1-aza-1,5,9-cyclododecatrien. Nach der Destillation erhält man 265 g (0,905 Mol) 3,3-Diäthyl-12-(3-pentyl)-1-aza-caclododecen; Sdp. 95° C/4 Pa.

c) Es wird wie in Beispiel 2b) beschrieben vorgegangen, jedoch unter Verwendung von 320 g (1,09 Mol) 3,3-Diäthyl-12-(3-pentyl)-1-aza-caclododecen, 89,5 g (0,546 Mol) Hydroxylaminsulfat, 110 g 37%iger Salzsäure und 400 ml Wasser. Nach der Neutralisation mit festem Natriumhydroxid erhält man 350 g (1,07 Mol) 2,2-Diäthyl-11-(3-pentyl)-11-amino-undecanal-oxim; $n_D^{20}$ = 1,4637.

d) Es wird wie in Beispiel 1c) beschrieben vorgegangen, jedoch unter Verwendung von 243 g (0,745 Mol) 2,2-Diäthyl-11-(3-pentyl)-11-amino-undecanal-oxim. Nach der Destillation erhält man 199 g (0,638 Mol) 1-(3-Pentyl)-10,10-diäthyl-1,11-diaminoundecan; Ausbeute 85,6% d. Th.; Sdp. 133–135° C/3 Pa; $n_D^{20}$ = 1,4704.

Analyse für $C_{20}H_{44}N_2$ (Molgewicht 312,59):

| | | | |
|---|---|---|---|
| berechnet | C 76,85 | H 14,19 | N 8,96% |
| gefunden | C 77,05 | H 13,92 | N 8,94% |

MS-Spektrum:
Molekül-Peak 312, Bruchstückmassen 283, 241, 224, 212, 128, 100.

$^1H$-NMR-Spektrum $\tau$ (ppm):
7,27 (m), 7,55 (s), 8,2–8,9 (m), 9,0–9,3 (m) im Verhältnis 1 : 2 : 25 : 16.

## Beispiel 10

a) Analog der in Beispiel 1a) beschriebenen Arbeitsweise werden 710 g (3,93 Mol) N-2-Methyl-pentyliden-(2-methylpenten-1-yl-amin) (hergestellt durch Umsetzung von 2-Methylvaleraldehyd mit Ammoniak gemäß US-Patentschrift 2 319 848) mit 432 g (8,0 Mol) 1,3-Butadien umgesetzt. Nach der Aufarbeitung des Reaktionsgemisches erhält man 995 g (3,45 Mol) 3-Methyl-3-n-propyl-12-(2-pentyl)-1-aza-1,5,9-cyclododecatrien als Isomerengemisch (2 Hauptisomere); Sdp 103–105° C/40 Pa; $n_D^{20}$ = 1,4886.

b) Es wird wie in Beispiel 2a) beschrieben vorgegangen, jedoch unter Verwendung von 289,5 g (1 Mol) 3-Methyl-3-n-propyl-12-(2-pentyl)-1-aza-1,5,9-cyclododecatrien. Nach der Destillation erhält man 263 g (0,896 Mol) 3-Methyl-3-n-propyl-12-(2-pentyl)-1-aza-caclododecen; Sdp. 125° C/53 Pa.

c) Es wird wie in Beispiel 2b) beschrieben vorgegangen, jedoch unter Verwendung von 293,55 g (1 Mol) 3-Methyl-3-n-propyl-12-(2-pentyl)-1-aza-caclododecen, 82,1 g (0,5 Mol) Hydroxylaminsulfat, 100 g 37%iger Salzsäure, 400 ml Wasser und 85 g festem Natriumhydroxid. Die sich abscheidende organische Phase enthält 325 g (0,996 Mol) 2-Methyl-2-n-propyl-11-(2-pentyl)-11-amino-undecanal-oxim; Ausbeute 99,6% d. Th.

d) Es wird wie in Beispiel 1c) beschrieben vorgegangen, jedoch unter Verwendung von 325 g (0,996 Mol) 2-Methyl-2-n-propyl-11-(2-pentyl)-11-amino-undecanal-oxim. Nach der Destillation erhält man 285 g (0,912 Mol) 1-(2-Pentyl)-10-methyl-10-n-propyl-1,11-diaminoundecan; Ausbeute 91,5% d. Th.; Sdp. 140–142° C/3 Pa; $n_D^{20}$ = 1,4665.

Analyse für $C_{20}H_{44}N_2$ (Molgewicht 312,59):

| | | | |
|---|---|---|---|
| berechnet | C 76,85 | H 14,19 | N 8,96% |
| gefunden | C 77,15 | H 14,52 | N 9,02% |

MS-Spektrum:
Molekül-Peak 312, Bruchstückmassen 283, 241, 224, 212, 128, 100.

$^1H$-NMR-Spektrum $\tau$ (ppm):
7,42 (m), 7,56 (s), 8,5–8,9 (m), 9,05–9,24 (m) im Verhältnis 1 : 2 : 25 : 16.

## Beispiel 11

a) Analog der in Beispiel 1a) beschriebenen Arbeitsweise werden 760 g (3,21 Mol) N-2-Äthyl-hexyliden-(2-äthylhexen-1-yl-amin) (hergestellt durch Umsetzung von 2-Äthylcapronaldehyd mit Ammoniak gemäß US-Patentschrift 2 319 848) mit 378 g (7 Mol) 1,3-Butadien umgesetzt. Nach der Aufarbeitung des Reaktionsgemisches erhält man 930 g (2,69 Mol) 3-Äthyl-3-n-butyl-12-(3-heptyl)-1-aza-1,5,9-cyclododecatrien als ein 7 : 3-Isomerengemisch; Sdp. 106–109° C/13 Pa; $n_D^{20}$ = 1,4895.

b) Es wird wie in Beispiel 2a) beschrieben vorgegangen, jedoch unter Verwendung von 396 g (1,15 Mol) 3-n-Butyl-3-äthyl-12-(3-heptyl)-1-aza-1,5,9-cyclododecatrien. Nach der Destillation erhält man 384 g (1,1 Mol) 3-n-Butyl-3-äthyl-12-(3-heptyl)-1-aza-cyclododecen; Sdp. 130° C/4 Pa.

c) Es wird wie in Beispiel 2b) beschrieben vorgegangen, jedoch unter Verwendung von 300 g (0,859 Mol) 3-n-Butyl-3-äthyl-12-(3-heptyl)-1-aza-cyclododecen, 70,3 g (0,43 Mol) Hydroxylaminsulfat und 85 g 37%iger Salzsäure sowie 400 ml Wasser. Nach der Neutralisation mit festem Natriumhydroxid erhält man 329 g (0,86 Mol) 2-n-Butyl-2-äthyl-11-(3-heptyl)-11-amino-undecanal-oxim; Ausbeute 100% d. Th.

d) Es wird wie in Beispiel 1c) beschrieben vorgegangen, jedoch unter Verwendung von 248 g (0,648 Mol) 2-n-Butyl-2-äthyl-11-(3-heptyl)-11-amino-undecanal-oxim. Nach der Destillation erhält man 195 g (0,53 Mol) 1-(3-Heptyl)-10-n-butyl-10-äthyl-1,11-diaminoundecan; Ausbeute 81,6% d. Th.; Sdp. 156 – 160° C/4 Pa; $n_D^{20}$ = 1,4672.

Analyse für $C_{24}H_{52}N_2$ (Molgewicht 368,69):

| | | | |
|---|---|---|---|
| berechnet | C 78,19 | H 14,22 | N 7,60% |
| gefunden | C 78,60 | H 14,44 | N 7,47% |

MS-Spektrum:
Molekül-Peak 368, Bruchstückmassen 339, 269, 240, 196, 128.

$^1$H-NMR-Spektrum $\tau$ (ppm):
7,39 (m), 7,56 (s), 8,5 – 8,9 (m), 9,05 (s) und 9,1 – 9,3 (m) im Verhältnis 1 : 2 : 33 : 16.

## Beispiel 12

a) Analog der in Beispiel 1a) beschriebenen Arbeitsweise werden 467 g (2,8 Mol) N-Propyliden-(2-äthyl-hexen-1-yl-amin) mit 324 g ( Mol) 1,3-Butadien umgesetzt. Nach einer Reaktionszeit von 4 Stunden bei 40° C erhält man nach der Aufarbeitung 624 g (2,27 Mol) 3,12-Diäthyl-3-n-butyl-1-aza-1,5,9-cyclododecatrien als Isomerengemisch; Sdp. 98 – 100° C/40 Pa; $n_D^{20}$ = 1,4905.

Das im obigen Beispiel verwendete N-Propyliden-(2-äthyl-hexen-1-yl-amin) wurde auf analoge Weise wie das N-Propyliden-(2-methylpropenylamin) hergestellt, jedoch unter Verwendung von 10 g Kalium-tert-butylat, 800 g (4,79 Mol) (2-Äthyl-hexyliden)-allylamin und 600 ml Tetrahydrofuran. Nach einer Reaktionszeit von 2 Stunden bei 35° C erhält man 682 g (4,08 Mol) N-Propyliden-(2-äthyl-hexen-1-yl-amin) als Isomerengemisch im Gewichtsverhältnis von 55 : 45; Sdp. 53 – 56° C/133 Pa; $n_D^{20}$ = 1,4698.

b) Es wird wie in Beispiel 2a) beschrieben vorgegangen, jedoch unter Verwendung von 275,5 g (1 Mol) 3-n-Butyl-3,12-diäthyl-1-aza-1,5,9-cyclododecatrien. Nach der Destillation erhält man 245,5 g (0,878 Mol) 3-n-Butyl-3,12-diäthyl-1-aza-cyclododecen; Sdp. 110° C/7 Pa.

c) Es wird wie in Beispiel 2b) beschrieben vorgegangen, jedoch unter Verwendung von 245,5 g (0,878 Mol) 3-n-Butyl-3,12-diäthyl-1-aza-cyclododecen, 74 g (0,45 Mol) Hydroxylaminsulfat, 100 g 37%iger Salzsäure und 200 ml Wasser. Nach der Neutralisation mit festem Natriumhydroxid erhält man 255 g (0,815 Mol) 2-n-Butyl-2,11-diäthyl-11-amino-undecanal-oxim.

d) Es wird wie in Beispiel 1c) beschrieben vorgegangen, jedoch unter Verwendung von 219 g (0,7 Mol) 2-n-Butyl-2,11-diäthyl-11-amino-undecanal-oxim. Nach der Destillation erhält man 164 g (0,55 Mol) 1,10-Diäthyl-10-n-butyl-1,11-diaminoundecan; Ausbeute 78,5% d. Th.; Sdp. 128 – 130° C/5 Pa; $n_D^{20}$ = 1,4630.

Analyse für $C_{19}H_{42}N_2$ (Molgewicht 298,56):

| | | | |
|---|---|---|---|
| berechnet | C 76,44 | H 14,18 | N 9,38% |
| gefunden | C 76,62 | H 13,93 | N 9,48% |

MS-Spektrum:
Molekül-Peak 298, Bruchstückmassen 269, 240, 226, 199.

$^1$H-NMR-Spektrum $\tau$ (ppm):
7,4 (m), 7,57 (s), 8,4 – 8,85 (m), 8,93 (s) und 9,95 – 9,3 (m) im Verhältnis 1 : 2 : 26 : 13.

## Beispiel 13

a) Analog der in Beispiel 1a) beschriebenen Arbeitsweise werden 678 g (3,31 Mol) N-Cyclohexyl-methyliden-(cyclohexyliden-methylamin) (hergestellt durch Umsetzung von Cyclohexanaldehyd mit Ammoniak; Sdp. 83° C/4 Pa; $n_D^{20}$ = 1,5260) mit 1,2-Butadien umgesetzt. Nach der Destillation erhält man 851 g (2,72 Mol) 3-Pentamethylen-12-cyclohexyl-1-aza-1,5,9-cyclodecatrien; Sdp. 140° C/3 Pa; $n_D^{20}$ = 1,5191.

b) Es wird wie in Beispiel 2a) beschrieben vorgegangen, jedoch unter Verwendung von 750 g (2,4 Mol) 3-Pentamethylen-12-cyclohexyl-1-aza-1,5,9-cyclododecatrien. Nach der Destillation erhält man 733,9 g (2,32 Mol) 3-Pentamethylen-12-cyclohexyl-1-aza-cyclododecen; Sdp. 140–142° C/3 Pa; n = 1,4982.

c) Es wird wie in Beispiel 2b) beschrieben vorgegangen, jedoch unter Verwendung von 317 g (1 Mol) 3-Pentamethylen-12-cyclohexyl-1-aza-cyclododecen, 70 g (1 Mol) Hydroxylaminhydrochlorid, 100 g 37%iger Salzsäure, 300 ml Wasser und 100 g festem Natriumhydroxid. Die sich abscheidende organische Phase enthält 340 g (0,97 Mol) 2-Pentamethylen-11-cyclohexyl-11-amino-undecanal-oxim; Ausbeute 97% d. Th.

d) Es wrid wie in Beispiel 1c) beschrieben vorgegangen, jedoch unter Verwendung von 340 g (0,97 Mol) 2-Pentamethylen-11-cyclohexyl-11-amino-undecanal-oxim. Nach der Destillation erhält man 252 g (0,75 Mol) 1-Cyclohexyl-10-pentamethylen-1,11-diaminoundecan; Ausbeute 77,2% d. Th., Sdp. 166–170° C/3 Pa; n = 1,4975.

Analyse für $C_{22}H_{44}N_2$ (Molgewicht 336,61):

| | | | |
|---|---|---|---|
| berechnet | C 78,50 | H 13,18 | N 8,32% |
| gefunden | C 78,76 | H 13,42 | N 8,17% |

MS-Spektrum:
Molekül-Peak 336, Bruchstückmassen 307, 290, 267, 253, 236, 224, 128, 112.

$^1$H-NMR-Spektrum $\tau$ (ppm):
7,48 (s) und 7,56 (m), 8,1 – 8,9 (m), 9,02 (s) im Verhältnis 3 : 37 : 4.

## Beispiel 14

a) Analog der in Beispiel 1a) beschriebenen Arbeitsweise werden 1010 g (6,35 Mol) N-Benzyliden-(2-methylpropenylamin) (hergestellt durch Umsetzung von Benzaldehyd mit Methallylamin und anschließende Isomerisierung in Gegenwart von Kalium-tert-butylat; Sdp. 65–66° C/7 Pa; n = 1,5836) mit 1,3-Butadien umgesetzt. Nach einer Reaktionszeit von 2 Stunden bei 85° C und anschließender Destillation erhält man 1398 g (5,24 Mol) 3,3-Dimethyl-12-phenyl-1-aza-1,5,9-cyclododecatrien; Sdp. 128–130° C/4 Pa; Smp. 66–68° C.

b) Es wird wie in Beispiel 1b) beschrieben vorgegangen, jedoch unter Verwendung von 289,3 g (1,09 Mol) 3,3-Dimethyl-12-phenyl-1-aza-1,5,9-cyclododecatrien, 88,7 g (0,54 Mol) Hydroxylaminsulfat, 100 g 37%iger Salzsäure, 400 ml Wasser und 100 g festem Natriumhydroxid. Die sich abscheidende organische Phase enthält 320 g (1,065 Mol) 2,2-Dimethyl-11-phenyl-11-amino-undeca-4,8-dienal-oxim; Ausbeute 97,9% d. Th.

c) Es wird wie in Beispiel 1c) beschrieben vorgegangen, jedoch unter Verwendung von 320 g (1,065 Mol) 2,2-Dimethyl-11-phenyl-11-amino-undeca-4,8-dienal-oxim. Nach der Destillation erhält man 296 g (1,02 Mol) 1-Phenyl-10,10-dimethyl-1,11-diaminoundecan; Ausbeute 95,8% d. Th.; Sdp. 150° C/3 Pa; n = 1,5054.

Analyse für $C_{19}H_{34}N_2$ (Molgewicht 290,50):

| | | | |
|---|---|---|---|
| berechnet | C 78,56 | H 11,80 | N 9,64% |
| gefunden | C 76,61 | H 11,97 | N 9,76% |

MS-Spektrum:
Molekül-Peak 290, Bruchstückmassen 261, 244, 213, 188, 106, 91.

$^1$H-NMR-Spektrum $\tau$ (ppm):
2,76 (s), 6,13 (t), 7,56 (s), 8,3 (m), 8,7 (s) und 8,78 (s), 9,13 (s) im Verhältnis 5 : 1 : 2 : 2 : 18 : 6.

## Beispiel 15

a) Analog Beispiel 1a) werden 629 g (3,55 Mol) N-Cyclopentyl-methyliden-(cyclopentylidenmethylamin) (hergestellt durch Umsetzung von Cyclopentanaldehyd mit Ammoniak; Sdp. 125° C/ $1,86 \times 10^3$ Pa; n = 1,5245) mit 1,3-Butadien umgesetzt. Nach der Destillation erhält man 798 g (2,8 Mol) 3-Tetramethylen-12-cyclopentyl-1-aza-1,5,9-cyclododecatrien; Sdp. 120° C/1 Pa.

b) Es wird wie in Beispiel 2a) beschrieben vorgegangen, jedoch unter Verwendung von 500 g (1,75 Mol) 3-Tetramethylen-12-cyclopentyl-1-aza-1,5,9-cyclododecatrien. Nach der Destillation erhält man 470 g (1,63 Mol) 3-Tetramethylen-12-cyclopentyl-1-aza-cyclododecen; Sdp. 130° C/7 Pa.

c) Es wird wie in Beispiel 2b) beschrieben vorgegangen, jedoch unter Verwendung von 470 g (1,63 Mol) 3-Tetramethylen-12-cyclopentyl-1-aza-cyclododecen, 133 g (0,81 Mol) Hydroxylaminsulfat, 162 g 37%iger Salzsäure und 400 ml Wasser. Nach der Neutralisation mit festem Natriumhydroxid erhält man 525 g (1,63 Mol) 2-Tetramethylen-11-cyclopentyl-11-amino-undecanal-oxim; Ausbeute 100% d Th.

d) Es wird wie in Beispiel 1c) beschrieben vorgegangen, jedoch unter Verwendung von 525 g (1,63 Mol) 2-Tetramethylen-11-cyclopentyl-11-amino-undecanal-oxim. Nach der Destillation erhält man 432 g (1,405 Mol) 1-Cyclopentyl-10-tetramethylen-1,11-diaminoundecan; Ausbeute 86,1% d. Th.; Sdp. 166 – 168° C/5 Pa; n =1,4922.

Analyse für $C_{20}H_{40}N_2$ (Molgewicht 308,55):

| | | | |
|---|---|---|---|
| berechnet | C 77,85 | H 13,07 | N 9,08% |
| gefunden | C 77,91 | H 13,37 | N 9,00%. |

MS-Spektrum:
Molekül-Peak 308, Bruchstückmassen 290, 279, 262, 239, 222, 210, 98.

$^1$H-NMR-Spektrum $\tau$ (ppm):
7,50 (m), 8,1 – 8,8 (m), 8,93 (s) im Verhältnis 3 : 33 : 4.

## Beispiel 16

78,6 (0,285 Mol) des gemäß Beispiel 5 hergestellten 1-Phenyl-10-methyl-1,11-diaminoundecans werden zusammen mit 400 ml Methanol und 8 g eines Ruthenium/Kohle-Katalysators (10 Gew.-% Ruthenium) in einen Stahlautoklav eingefüllt und während 5 Stunden bei 120°C und einem Wasserstoffdruck von 100 bar hydriert. Nach der Aufarbeitung des Reaktionsgemsiches erhält man 42,7 g (0,152 Mol) 1-Cyclohexyl-10-methyl-1,11-diaminoundecan; Ausbeute 53,3% d. Th.; Sdp. 136 – 138° C/4 Pa; $n_D^{20}$ = 1,4809.

Analyse für $C_{18}H_{38}N_2$ (Molgewicht 282,52):

| | | | |
|---|---|---|---|
| berechnet | C 76,53 | H 13,56 | N 9,92% |
| gefunden | C 76,39 | H 13,38 | N 9,58% |

$^1$H-NMR-Spektrum $\tau$ (ppm):
7,5 (m), 8,1 – 8,7 (m) und 8,84 (s), 9,07 (d) im Verhältnis 3 : 32 : 3.

## Beispiel 17

Nach der in Beispiel 16 beschriebenen Arbeitsweise werden 143 g (0,493 Mol) 1-Phenyl-10,10-dimethyl-1,11-diaminoundecan hydriert. Nach der Destillation erhält man 95 g (0,321 Mol) 1-Cyclohexyl-10,10-dimethyl-1,11-diaminoundecan; Ausbeute 65% d. Th.; Sdp. 147° C/4 Pa; $n_D^{20}$ = 1,4805.

Analyse für $C_{19}H_{40}N_2$ (Molgewicht 296,54):

| | | | |
|---|---|---|---|
| berechnet | C 76,96 | H 13,60 | N 9,45% |
| gefunden | C 77,08 | H 13,87 | N 9,32% |

MS-Spektrum:
Molekül-Peak 296, Bruchstückmassen 281, 267, 250, 227, 213, 196, 184, 128, 112.

$^1$H-NMR-Spektrum $\tau$ (ppm):
7,5 (m) und 7,59 (s), 8,1 – 8,9 (m), 9,02 (s), 9,14 (s) im Verhältnis 3 : 27 : 4 : 6.

## Beispiel 18

a) Es wird wie in Beispiel 1a) beschrieben vorgegangen, jedoch unter Verwendung von 302,5 g (2 Mol) N-Cyclohexyliden-(2-methylpropenylamin) und 250 g (4,62 Mol) 1,3-Butadien. Durch Destillation erhält man 382 g (1,48 Mol) 3,3-Dimethyl-12-pentamethylen-1-aza-1,5,9-cyclododecatrien; Sdp. 96° C/4 Pa; $n_D^{20}$ = 1,5116. Das N-Cyclohexyliden-(2-methylpropenylamin) wurde aus Cyclohexanon und Methallylamin und anschließende Isomerisierung des Reaktionsproduktes mit Kalium-tert.-butylat hergestellt; Sdp. 96° C/1700 Pa; $n_D^{20}$ = 1,5160.

b) Es wird wie in Beispiel 1b) beschrieben vorgegangen, jedoch unter Verwendung von 259,5 g (1 Mol) 3,3-Dimethyl-12-pentamethylen-1-aza-1,5,9-cyclododecatrien, 69,5 g (1 Mol) Hydroxylaminhydrochlorid, 20 g 37%iger Salzsäure und 250 ml Wasser. Nach der Aufarbeitung erhält man 219,3 g 2,2-Dimethyl-11-pentamethylen-11-amino-undeca-4,8-dienal-oxim; Ausbeute 74,5% d. Th.; $n_D^{20}$ = 1,5117.

c) Es wird wie in Beispiel 1c) beschrieben vorgegangen, jedoch unter Verwendung von 160 g (0,545 Mol) 2,2-Dimethyl-11-pentamethylen-11-amino-undeca-4,8-dienal-oxim. Nach der Destillation erhält man 127 g (0,45 Mol) 1-Pentamethylen-10,10-diäthyl-1,11-diaminoundecan; Ausbeute 82,5% d. Th.; Sdp. 112° C/4 Pa; $n_D^{20}$ = 1,4833.

Analyse für $C_{18}H_{38}N_2$ (Molgewicht 282,52):

| | | | |
|---|---|---|---|
| berechnet | C 76,53 | H 13,56 | N 9,92% |
| gefunden | C 76,71 | H 13,33 | N 9,81% |

MS-Spektrum:
Molekül-Peak 282, Bruchstückmassen 253, 239, 208, 180, 126, 98.

$^1$H-NMR-Spektrum $\tau$ (ppm):
7,58 (s), 8,4 – 8,8 (m), 8,94 (s), 9,13 (s) im Verhältnis 2 : 26 : 4 : 6.

Beispiel 19

a) Es wird wie in Beispiel 1a) beschrieben vorgegangen, jedoch unter Verwendung von 94,2 g (0,564 Mol) N-Heptyliden-(2-methylpropenylamin) [1-n-Hexyl-4,4-dimethyl-2-aza-1,3-butadien] und 80 g (1,48 Mol) 1,3-Butadien. Durch Destillation erhält man 113 g (0,41 Mol) 3,3-Dimethyl-12-n-hexyl-1-aza-1,5,9-cyclododecatrien; Sdp. 100° C/4 Pa; $n_D^{20}$ = 1,4841.

Das N-Heptyliden-(2-methylpropenylamin) wurde aus Heptanal und Methallylamin und durch anschließende Isomerisierung des Reaktionsproduktes mit Kalium-tert.-butylat hergestellt; Sdp. 54° C/5 Pa; $n_D^{20}$ = 1,4662.

b) Es wird wie in Beispiel 1b) beschrieben vorgegangen, jedoch unter Verwendung von 113 g (0,41 Mol) 3,3-Dimethyl-12-hexyl-1-aza-1,5,9-cyclododecatrien, 33 g (0,202 Mol) Hydroxylaminsulfat, 50 g konz. Salzsäure und 250 ml Wasser. Nach der Aufarbeitung erhält man 125 g (0,405 Mol) 2,2-Dimethyl-11-n-hexyl-11-amino-undeca-4,8-dienal-oxim; Ausbeute 99% d. Th.

c) Es wird wie in Beispiel 1c) beschrieben vorgegangen, jedoch unter Verwendung von 116 g (0,377 Mol) 2,2-Dimethyl-11-n-hexyl-11-amino-undeca-4,8-dienal-oxim. Nach der Destillation erhält man 78 g (0,262 Mol) 1-n-Hexyl-10,10-dimethyl-1,11-diaminoundecan; Ausbeute 69,5% d. Th.; Sdp. 135° C/4 Pa; $n_D^{20}$ = 1,4624.

Analyse für $C_{19}H_{42}N_2$ (Molgewicht 298,56):

| | | | |
|---|---|---|---|
| berechnet | C 76,44 | H 14,18 | N 9,38% |
| gefunden | C 76,61 | H 14,02 | N 9,22% |

MS-Spektrum:
Molekül-Peak 298, Bruchstückmassen 283, 269, 213, 196, 184, 128, 114.

$^1$H-NMR-Spektrum $\tau$ (ppm):
7,35 (m), 7,57 (s), 8,5 – 8,8 (m), 8,92 (s), 9,13 (m) im Verhältnis 1 : 2 : 26 : 4 : 9.

Beispiel 20

a) Es wird wie in Beispiel 1a) beschrieben vorgegangen, jedoch unter Verwendung von 222,4 g (2,0 Mol) N-Isopropyliden-(2-methylpropenylamin). Durch Destillation erhält man 300 g (1,37 Mol) 3,3,12,12-Tetramethyl-1-aza-1,5,9-cyclododecatrien; Sdp. 58° C/4 Pa; $n_D^{20}$ = 1,4858. Das N-Isopropyliden-(2-methylpropenylamin) wurde aus Aceton und Methallylamin und durch anschließende Isomerisierung des Reaktionsproduktes hergestellt; Sdp. 89 – 90° C; $n_D^{20}$ = 1,4762.

b) Es wird wie in Beispiel 1b) beschrieben vorgegangen, jedoch unter Verwendung von 110 g (0 5 Mol) 3,3,12,12-Tetramethyl-1-aza-1,5,9-cyclododecatrien, 41 g (0,25 Mol) Hydroxylaminsulfat, 50 ml konz. Salzsäure und 250 ml Wasser. Nach der Destillation erhält man 85 g (0,377 Mol) 2,2,11,11-Tetramethyl-11-amino-undeca-4,8-dienal-oxim; Ausbeute 67,4% d. Th.; Sdp. 130° C/7 Pa.

c) Es wird wie in Beispiel 1c) beschrieben vorgegangen, jedoch unter Verwendung von 126 g (0,5 Mol) 2,2,11,11-Tetramethyl-11-amino-undeca-4,8-dienal-oxim. Nach der Destillation erhält man 91 g (0,377 Mol) 1,1,10,10-Tetramethyl-1,11-diaminoundecan; Ausbeute 75,3% d. Th.; Sdp. 92° C/5 Pa; $n_D^{20}$ = 1,4590.

Analyse für $C_{15}H_{34}N_2$ (Molgewicht 242,45):

| | | | |
|---|---|---|---|
| berechnet | C 74,31 | H 14,14 | N 11,56% |
| gefunden | C 74,47 | H 14,15 | N 11,57% |

MS-Spektrum:
Molekül-Peak 242, Bruchstückmassen 227, 210, 196, 140, 97.

$^1$H-NMR-Spektrum $\tau$ (ppm):
7,58 (s), 8,6 – 8,8 (m), 8,88 und 8,89 (s), 9,12 (s) im Verhältnis 2 : 16 : 10 : 6.

Beispiel 21

a) Es wird wie in Beispiel 1a) beschrieben vorgegangen, jedoch unter Verwendung von 93,6 g (0,5 Mol) N-Benzyliden-(2-äthyl-1-butenylamin) (1-Phenyl-4,4-diäthyl-2-aza-1,3-butadien, hergestellt aus Benzylamin und 2-Äthyl-butenyl und durch anschließende Isomerisierung des Reaktionsproduktes in Gegenwart von Kalium-tert.-butylat; Sdp. 70° C/7 Pa; $n_D^{20}$ = 1,5598; vgl. J. Org. Chem., 43, Nr. 4, 782-84 [1978]). Nach der Destillation erhält man 116 g (0,393 Mol) 3,3-Diäthyl-12-phenyl-1-aza-1,5,9-cyclododecatrien; Sdp. 105° C/4 Pa; $n_D^{20}$ = 1,5369.

b) Es wird wie in Beispiel 1b) beschrieben vorgegangen, jedoch unter Verwendung von 95,3 g (0,323 Mol) 3,3-Diäthyl-12-phenyl-1-aza-1,5,9-cyclododecatrien, 20 g 37%iger Salzsäure, 22,4 g (0,322 Mol) Hydroxylaminhydrochlorid und 250 ml Wasser. Nach der Aufarbeitung erhält man 103,1 g (0,314 Mol) 2,2-Diäthyl-11-phenyl-11-amino-undeca-4,8-dienal-oxim; Ausbeute 97,5% d. Th.

c) Es wird wie in Beispiel 1c) beschrieben vorgegangen, jedoch unter Verwendung von 103 g (0,314 Mol) 2,2-Diäthyl-11-phenyl-11-amino-undeca-4,8-dienal-oxim. Nach Destillation erhält man 76 g (0,239 Mol) 1-Phenyl-10,10-diäthyl-1,11-diaminoundecan; Ausbeute 76% d. Th.; Sdp. 146° C/2 Pa; $n_D^{20}$ = 1,5090.

Analyse für $C_{21}H_{38}N_2$ (Molgewicht 318,55):

| | | | |
|---|---|---|---|
| berechnet | C 79,18 | H 12,03 | N 8,79% |
| gefunden | C 79,45 | H 12,08 | N 8,77% |

MS-Spektrum:
Molekül-Peak 318, Bruchstückmassen 289, 272, 213, 188, 131, 117, 106, 91.

$^1$H-NMR-Spektrum $\tau$ (ppm):
2,72 (s), 6,16 (t), 7,58 (s), 8,35 (m), 8,6 – 8,9 (m), 9,21 (t) im Verhältnis 5 : 1 : 2 : 2 : 22 : 6.

Beispiel 22

a) Es wird wie in Beispiel 1a) beschrieben vorgegangen, jedoch unter Verwendung von 215,3 g (1 Mol) N-Benzyliden-(2-äthyl-1-hexenylamin) (1-Phenyl-4-äthyl-4-n-butyl-2-aza-1,3-butadien; hergestellt durch Umsetzung von Benzylamin mit 2-Äthyl-hexenal und anschließende Isomerisierung des Reaktionsproduktes mit Kalium-tert.-butylat; Sdp: 90° C/7 Pa; n = 1,5630). Nach der Destillation erhält man 288 g (0,891 Mol) 3-n-Butyl-3-äthyl-12-phenyl-1-aza-1,5,9-cyclododecatrien; Sdp. 130° C/2 Pa; n = 1,5296.

b) Es wird wie in Beispiel 1b) beschrieben vorgegangen, jedoch unter Verwendung von 283,4 g (0,875 Mol) 3-n-Butyl-3-äthyl-12-phenyl-1-aza-1,5,9-cyclododecatrien, 60,8 g (0,87 Mol) Hydroxylaminhydrochlorid, 50 ml konz. Salzsäure und 250 ml Wasser. Nach der Aufarbeitung erhält man 310 g (0,87 Mol) 2-n-Butyl-2-äthyl-11-phenyl-11-aminoundeca-4,8-dienal-oxim; Ausbeute 99,5% d. Th.

c) Es wird wie in Beispiel 1c) beschrieben vorgegangen, jedoch unter Verwendung von 310 g (0,87 Mol) 2-n-Butyl-2-äthyl-11-phenyl-11-amino-undeca-4,8-dienal-oxim. Nach der Destillation erhält man 205 g (0,593 Mol) 1-Phenyl-10-n-butyl-10-äthyl-1,11-diaminoundecan; Ausbeute 68,1% d. Th.; Sdp. 155 – 158° C/5 Pa; n = 1,5045.

Analyse für $C_{23}H_{42}N_2$ (Molgewicht 346,60):

| | | | |
|---|---|---|---|
| berechnet | C 79,70 | H 12,21 | N 8,08% |
| gefunden | C 79,48 | H 12,46 | N 8,29% |

MS-Spektrum:
Molekül-Peak 346, Bruchstückmassen 317, 300, 188, 106, 91.

$^1$H-NMR-Spektrum $\tau$ (ppm):
2,73 (s), 6,18 (t), 7,58 (s), 8,35 (m), 8,6 – 8,9 (m), 9,08 und 9,22 (t) im Verhältnis 5 : 1 : 2 : 2 : 26 : 6.

Beispiel 23

a) Es wird wie in Beispiel 1a) beschrieben vorgegangen, jedoch unter Verwendung von 184 g (1,34 Mol) N-(2-Äthyl)-buten-2-yliden-propenylamin (hergestellt durch Umsetzung von 2-Äthyl-butenal und Allylamin und anschließende Isomerisierung des Reaktionsproduktes analog Zhurnal Organicheskoi Khimii, 6, Nr. 11, 2197-9 [1970]; Sdp. 70° C/1700 Pa; n Δ1,5227). Nach der Destillation erhält man 295 g (1,21 Mol) 3-Methyl-12-(3-penten-2-yl)-1-aza-1,5,9-cyclododecatrien; Sdp. 100° C/4 Pa; n =1,5056.

b) Es wird wie in Beispiel 1b) beschrieben vorgegangen, jedoch unter Verwendung von 122,7 g (0,5 Mol) 3-Methyl-12-(3-penten-2-yl)-1-aza-1,5,9-cyclododecatrien, 41,1 g (0,25 Mol) Hydroxylaminsulfat, 50 ml konz. Salzsäure und 250 ml Wasser. Nach der Aufarbeitung erhält man 135,9 g (0,488 Mol) 2-Methyl-11-(3-penten-2-yl)-11-aminoundeca-4,8-dienal-oxim; Ausbeute 97,6% d. Th.; n =1,5091.

c) Es wird wie in Beispiel 1c) beschrieben vorgegangen, jedoch unter Verwendung von 135 g (0,485 Mol) 2-Methyl-11-(3-penten-2-yl)-11-amino-undeca-4,8-dienal-oxim. Nach der Destillation erhält man 95 g (0,352 Mol) 1-(3-Pentyl)-10-methyl-1,11-diaminoundecan; Ausbeute 72,5% d. Th.; Sdp. 115° C/5 Pa; n =1,4662.

Analyse für $C_{17}H_{38}N_2$ (Molgewicht 270,51):

| | | | |
|---|---|---|---|
| berechnet | C 75,48 | H 14,16 | N 10,36% |
| gefunden | C 75,50 | H 14,11 | N 10,41% |

MS-Spektrum:
Molekül-Peak 270, Bruchstückmassen 241, 199, 182, 170, 100.

$^1$H-NMR-Spektrum $\tau$ (ppm):
7,2 – 7,6 (m), 7,68 (s), 7,85 (s), 9,1 (m) im Verhältnis 3 : 22 : 4 : 9.

Beispiel 24

a) Es wird wie in Beispiel 1a) beschrieben vorgegangen, jedoch unter Verwendung von 105 g (0,765 Mol) N-(2-Äthyl)-buten-2-yliden-propenylamin und 120 g (1,76 Mol) Isopren. Nach einer Reaktionszeit von 5 Stunden bei 90° C erhält man bei der anschließenden Destillation 85 g (0,312 Mol) 3,5- (bzw. 6), 9- (bzw. 10)Trimethyl-12-(3-penten-2-yl)-1-aza-1,5,9-cyclododecatrien; Sdp. 108 – 110° C/5 Pa; n =1,5078.

b) Es wird wie in Beispiel 1b) beschrieben vorgegangen, jedoch unter Verwendung von 83,75 g (0,305 Mol) des obigen 1-Aza-1,5,9-cyclododecatriens, 26,8 g (0,163 Mol) Hydroxylaminsulfat, 35 ml konz. Salzsäure und 250 ml Wasser. Nach der Aufarbeitung erhält man 90 g (0,294 Mol) 2,4(5),8(9)-Trimethyl-11-(3-penten-2-yl)-11-amino-undeca-4,8-dienal-oxim; Ausbeute 96,5% d. Th.

c) Es wird wie in Beispiel 1c) beschrieben vorgegangen, jedoch unter Verwendung von 35 g (0,117 Mol) 2,4(5),8(9)-Trimethyl-11-(3-penten-2-yl)-11-amino-undeca-4,8-dienal-oxim. Nach der Destillation erhält man 33,5 g (0,112 Mol) das 1-(3-Phenyl)-3(4),7(8),10-trimethyl-1,11-diaminoundecan; Ausbeute 95,5% d.Th.; Sdp. 117° C/2 Pa; n =1,4731.

Analyse für $C_{19}H_{42}N_2$ (Molgewicht 298,56):

| | | | |
|---|---|---|---|
| berechnet | C 76,44 | H 14,18 | N 9,38% |
| gefunden | C 76,5 | H 14,0 | N 9,2% |

MS-Spektrum:
kein Molekül-Peak, Bruchstückmassen 269, 227, 210, 198, 100.

$^1$H-NMR-Spektrum $\tau$ (ppm):
7,0 – 7,65 (m), 8,2 – 8,95 (m), 8,95 – 9,2 (m) im Verhältnis 3 : 24 : 15.

Beispiel 25

a) Es wird wie in Beispiel 1b) beschrieben vorgegangen, jedoch unter Verwendung von 23,3 g (0,1 Mol 3,3-Dimethyl-12-isopropyl-1-aza-1,5,9-cyclododecatrien, 20 g 37%iger Salzsäure, 20 ml Wasser und 10,8 g (0,1 Mol) Phenylhydrazin. Nach der Aufarbeitung wie in Beispiel 1b) beschrieben, erhält man 34,1 g (0,1 Mol) 2,2-Dimethyl-11-isopropyl-11-amino-undeca-4,8-dienal-phenylhydrazon; Ausbeute 100% d. Th.

Analyse für $C_{22}H_{35}N_3$ (Molgewicht 341,54):

| | | | |
|---|---|---|---|
| berechnet | C 77,37 | H 10,33 | N 12,30% |
| gefunden | C 77.03 | H 10.58 | N 12,23% |

MS-Spektrum:
Molekül-Peak 341, Bruchstückmassen 298, 270, 234, 161, 92, 72.

$^1$H-NMR-Spektrum $\tau$ (ppm):
2,65–3,3 (m), 4,56 (m), 7,45 (dt), 7,87 (m), 8,03 (s), 8,35 (m), 8,88 (s), 9,06 (dd) im Verhältnis 7 : 4 : 1 : 8 : 1 : 6 : 6.

b) Es wird wie in Beispiel 1c) beschrieben vorgegangen, jedoch unter Verwendung von 34,1 g (0,1 Mol) 2,2-Dimethyl-11-isopropyl-11-amino-undeca-4,8-dienal-phenylhydrazon. Nach einer Reaktionszeit von 15 Stunden bei 100°C unter einem Wasserstoffdruck von 100 bar und anschließender Aufarbeitung erhält man 19,5 g (0,076 Mol) 1-Isopropyl-10,10-dimethyl-1,11-diaminoundecan; Ausbeute 76% d. Th.

## Beispiel 26

a) Es wird wie in Beispiel 1b) beschrieben vorgegangen, jedoch unter Verwendung von 46,6 g (0,2 Mol) 3,3-Dimethyl-12-isopropyl-1-aza-1,5,9-cyclododecatrien, 40 g 37%iger Salzsäure, 40 ml Wasser und 5 g (0,1 Mol) Hydrazinhydrat. Nach der Aufarbeitung erhält man 42 g (0,84 Mol) Di-(2,2-dimethyl-11-isopropyl-11-aminoundeca-4,8-dienal)-hydrazon; Ausbeute 84% d. Th.

Analyse für $C_{32}H_{58}N_4$ (Molgewicht 498,84):

| | | | |
|---|---|---|---|
| berechnet | C 77,05 | H 11,72 | N 11,23% |
| gefunden | C 77,83 | H 12,08 | N 10,84% |

MS-Spektrum:
Molekül-Peak 498, Bruchstückmassen 483, 455, 438, 426, 372, 318, 276.

$^1$H-NMR-Spektrum $\tau$ (ppm):
2,38 (s), 4,58 (m), 7,5 (m), 7,88 (m), 8,1–8,7 (m), 8,88 (s), 9,06 (dd) im Verhältnis 2 : 8 : 2 : 16 : 6 : 12 : 12.

b) Es wird wie in Beispiel 1c) beschrieben vorgegangen, jedoch unter Verwendung von 42 g (0,084 Mol) Di-(2,2-dimethyl-11-isopropyl-11-amino-undeca-4,8-dienal)-hydrazin. Nach der Destillation erhält man 26 g (0,102 Mol) 1-Isopropyl-10,10-dimethyl-diaminoundecan; Ausbeute 60,6% d. Th.

## Beispiel 27

a) Es wird wie in Beispiel 1b) beschrieben vorgegangen, jedoch unter Verwendung von 23,3 g (0,1 Mol) 3,3-Dimethyl-12-isopropyl-1-aza-1,5,9-cyclododecatrien, 20 g 37%iger Salzsäure, 20 ml Wasser und 11,15 g (0,1 Mol) Semicarbazidhydrochlorid. Nach der Aufarbeitung erhält man 26,5 g (0,086 Mol) 2,2-Dimethyl-11-isopropyl-11-aminoundeca-4,8-dienal-semicarbazon; Ausbeute 86% d. Th.

Analyse für $C_{17}H_{32}H_4O$ (Molgewicht 308,47):

| | | | | |
|---|---|---|---|---|
| berechnet | C 66,19 | H 10,46 | N 18,16 | O 5,19% |
| gefunden | C 68,5 | H 10,5 | N 17,4 | O 5,0% |

MS-Spektrum:
Molekül-Peak 308, Bruchstückmassen 265, 248, 205, 182, 129, 72.

$^1$H-NMR-Spektrum $\tau$ (ppm):
0,9 (m), 2,98 (s), 4,32 (s), 4,58 (m), 7,48 (dt), 7,9 (m), 8,1–8,8 (m), 8,91 (s), 9,06 (dd) im Verhältnis 1 : 1 : 2 : 4 : 1 : 8 : 3 : 6 : 6.

b) Es wird wie in Beispiel 1c) beschrieben vorgegangen, jedoch unter Verwendung von 26,5 g (0,086 Mol) 2,2-Dimethyl-11-isopropyl-11-amino-undeca-4,8-dienal-semicarbazon. Nach der Destillation erhöht man 13,8 g (0,54 Mol) 1-Isopropyl-10,10-dimethyl-1,11-diaminoundecan; Ausbeute 62,6% d. Th.

## Beispiel 28

a) Es wird wie in Beispiel 2b) beschrieben vorgegangen, jedoch unter Verwendung von 68,5 g (0,5 Mol) 3,3-Dimethyl-12-isopropyl-1-aza-cyclododecen, 50 g 37%ige Salzsäure; 54 g (0,5 Mol) Benzylamin und 200 ml Wasser. Nach der Neutralisation mit 22 g (0,55 Mol) festem Natriumhydroxid erhält man 97 g (0,282 Mol) 2,2-Dimethyl-11-isopropyl-11-amino-undecanal-benzylamin; Ausbeute 56,4% d. Th.

Analyse für $C_{23}H_{40}N_2$ (Molgewicht 344, 59):

| | | | |
|---|---|---|---|
| berechnet | C 80,17 | H 11,70 | N 8,13% |
| gefunden | C 80,0 | H 12,1 | N 7,7% |

MS-Spektrum:
Molekül-Peak 344, Bruchstückmassen 301, 253, 161, 91.

$^1$H-NMR-Spektrum $\tau$ (ppm):
2,7 (m), 6,18 (s), 7,3–9,2 (m) im Verhältnis 6 : 2 : 32.

b) Es wird wie in Beispiel 1c) beschrieben vorgegangen, jedoch unter Verwendung von 97 g (0,282 Mol) 2,2-Dimethyl-11-isopropyl-11-amino-undecanal-benzylamin und eines Platinkatalysators. Man hydriert 10 Stunden bei 100°C unter einem Wasserstoffdruck von 100 bar. Nach der Aufarbeitung erhält man 52 g (0,203 Mol) 1-Isopropyl-10,10-dimethyl-1,11-diaminoundecan; Ausbeute 72% d. Th.

Beispiel 29

116,7 g (0,5 Mol) 3,3-Dimethyl-12-isopropyl-1-aza-1,5,9-cyclododecatrien werden innerhalb von 20 Minuten zu einem Gemisch aus 250 ml Isopropanol, 30 ml und 55 g (0,56 Mol) Schwefelsäure getropft. Dann wird noch eine Stunde unter Rückfluß gekocht, und das Gemisch wird bei einem Wasserstoffdruck von 150 bar und einer Temperatur von 120°C in einen mit 200 g flüssigem Ammoniak und 500 ml Methanol gefüllten Autoklav eindosiert. Nach einer Reaktionszeit von 15 Stunden kühlt man ab und läßt den überschüssigen Ammoniak und den Wasserstoff abblasen. Bei der anschließenden Destillation im Hochvakuum erhält man 93 g (0,364 Mol) 1-Isopropyl-10,10-dimethyl-1,11-diaminoundecan; Ausbeute 72,5% d. Th.

Anwendungsbeispiele

A) Herstellung von Polyamiden

Beispiel I

In einem Kolben, der mit Rührer, Tropftrichter und Rückflußkühler versehen ist, werden 54,5 g Terephthalsäure in einem Gemisch von 750 ml Äthanol und 750 ml Wasser aufgeschlämmt und zum Rückfluß erhitzt. Nun werden aus dem Tropftrichter 103,2 g 1-(3-Pentyl)-10,10-diäthyl-1,11-diaminoundecan dazugegeben. Nach 20 Minuten wird langsam auf Raumtemperatur (20–25°C) abgekühlt, und das ausgefallene Salz wird abfiltriert. Nach dem Trocknen im Vakuum erhält man 147 g Salz (93% d. Th.). 10 g dieses Salzes werden unter Stickstoff in ein Bombenrohr eingeschmolzen und eine Stunde in einem Salzbad auf 270°C erhitzt. Das Salz schmilzt dabei zu einer farblosen Schmelze. Nach dem Abkühlen auf Raumtemperatur wird die erstarrte Schmelze aus dem Bombenrohr entnommen und in einer offenen Polykondensationsapparatur unter Ausschluß von Luft und ständigem Durchleiten von Stickstoff 6 Stunden bei 270°C gehalten. Beim Abkühlen erstarrt die viskose Schmelze zu einer glasklaren, farblosen Masse. Die reduzierte Lösungsviskosität des erhaltenen Polyamids, gemessen als 0,5%ige Lösung in m-Kresol bei 25°C, beträgt 0,91 dl/g; Glasumwandlungstemperatur, bestimmt im Differentialkalorimeter (DSC) = 123°C.

Eine bei 270°C mittels einer hydraulischen Presse hergestellte Folie wird bei Raumtemperatur einer relativen Luftfeuchtigkeit von 65% ausgesetzt, bis keine Gewichtszunahme mehr festzustellen ist. Der Sättigungswert beträgt 0,7 Gewichtsprozent. Setzt man die Folie der Einwirkung von kochendem Wasser aus, so ist nach 6 Stunden noch keinerlei Beeinträchtigung der Transparenz festzustellen.

Beispiel II

In einem Rundkolben, der mit Rührer, Rückflußkühler und Tropftrichter versehen ist, werden 0,1 Mol Terephthalsäure in 300 ml 70%igem Äthanol zum Sieden erhitzt. In die kochende Suspension läßt man unter Rühren 0,1 Mol 1-Cyclohexyl-10-pentamethylen-1,11-diaminoundecan im Verlaufe von ca. 10 Minuten aus dem Tropftrichter einfließen und spült darin haftende Diaminreste mit etwas Äthanol quantitativ in das Reaktionsgemisch. Die dabei entstandene klare Lösung läßt man unter ständigem Rühren erkalten, filtriert das dabei ausgefallene Salz ab und trocknet es im Vakuum bei 90°C.

In ein Bombenrohr, welches mit einem Schraubdeckel mit eingebautem Überdruckventil ausgestattet ist, werden folgende Komponenten eingewogen:

2,15 g 4,4'-Diamino-3,3'-dimethyldicyclohexylmethan,

1,502 g Isophthalsäure,
8,535 g Sals aus 1-Cyclohexyl-10-pentamethylen-1,11-diaminoundecan und Terephthalsäure.

Nachdem man die Luft im Bombenrohr vollständig durch Stickstoff verdrängt hat, verschließt man das Bombenrohr und taucht es in ein Salzbad, dessen Temperatur 270°C beträgt. Nach 30–60 Minuten ist eine homogene, glasklare Schmelze entstanden. Nach insgesamt 3 Stunden wird die Vorkondensation abgebrochen, indem man das Bombenrohr aus dem Salzbad entfernt und den Überdruck durch Öffnen des Ventils abläßt. Das erstarrte glasklare Vorkondensat wird aus dem Bombenrohr entfernt und in ein Kondensationsgefäß übergeführt. Unter strengem Ausschluß von Luft und unter dauerndem Durchleiten von Stickstoff wird die Schmelze bei 280°C Salzbadtemperatur 5 Stunden polykondensiert, wobei das Reaktionswasser durch den Stickstoffstrom laufend entfernt wird. Beim Abkühlen erstarrt die Schmelze zu einer glasklaren Masse.

2–3 g des hergestellten Copolyamids werden in einer beheizbaren hydraulischen Presse bei 270°C zu einer Folie von ca. 0,4 mm bis 1 mm Dicke verpreßt. Zur Bestimmung der Wasseraufnahme wird die Folie bei Raumtemperatur einer relativen Luftfeuchtigkeit von 65% ausgesetzt, bis keine Gewichtszunahme mehr festzustellen ist. Der Sättigungswert beträgt 1,2 Gewichtsprozent. Die reduzierte Lösungsviskosität des Copolyamids, gemessen als 0,5%ige Lösung in m-Kresol bei 25°C, beträgt 1,09 dl/g; Glasumwandlungstemperatur, bestimmt im Differentialkalorimeter (DSC) = 166°C. Die Beständigkeit der Transparenz gegenüber kochendem Wasser ist sehr gut, das heißt, auch nach mehreren Tagen ist keine Beeinträchtigung der Transparenz des Copolyamids feststellbar.

## B) Verwendung als Härter für Epoxidharze

### Beispiele III bis XIV

Als Härter für Epoxidharze werden die nach den Beispielen 1, 2, 5, 6, 7, 14, 21 und 22 hergestellten Amine und zum Vergleich Trimethylhexamethylendiamin verwendet.

Die erfindungsgemäßen Diamine zeichnen sich durch geringe Eigenfarbe und eine niedrige Viskosität aus. Der durch das höhere Molekulargewicht erhöhte Härteranteil zusammen mit der niedrigen Viskosität der Amine führt zu einer günstigen Viskosität der zusammen mit dem Epoxidharz erhaltenen Mischung.

Ein überraschender Vorteil der neuen Amine ist das außerordentlich günstige Verhalten beim offenen Stehen an der Luft, wie aus den Versuchswerten der folgenden Tabelle 1 hervorgeht.

Tabelle 1

| | Härter, hergestellt nach Beispiel | | | | | | Trimethylhexa-methylendiamin |
|---|---|---|---|---|---|---|---|
| | 1 bzw. 2 | 7 | 5 bzw. 6 | 14 | 21 | 22 | |
| Viskosität 25°C (mPas · s) | 15 | <10 | 30 | 50 | 75 | 105 | <10 |
| Löslich in $H_2O$ (ja oder nein) | nein | ja, trübe | ja, warm | nein | nein | nein | ja |
| In Methylethylketon | ja | ja | ja | ja | ja | ja | ja |
| In Aethanol | ja | ja | ja | ja | ja | ja | ja |
| Gewichtszunahme nach 24 h bei Raumtemperatur (~5 g im Büchsendeckel offen stehen gelassen) | 6,1% | | 8% | 4,6% | 4,1% | 3,2% | 30% wurde viskos |

Als Reaktionspartner für die neuen Amine wird als typisches Epoxidharz ein flüssiger, unmodifizierter Bisphenol-A-Epikörper (Harz A) mit einer Viskosität bei 25° C von 10 000 cP und einem Epoxidgehalt von 5,35 Äq./Kg herangezogen.

Das Harz wird jeweils im stöchiometrischen Verhältnis bei 25° C mit dem zu prüfenden Amin sorgfältig gemischt. Ein geringer Teil der Mischung wird sofort zur Bestimmung der Gebrauchsdauer bei 40° abgezweigt, die als Zeit bis zum Anstieg der Viskosität der Mischung auf 3000 cP definiert ist.

Der Hauptteil der Mischung wird anschließend im Vakuum sorgfältig von der beim Mischen mit eingerührten Luft befreit und dann wie folgt verwendet:

a) Herstellung von Formstoffplatten zur Gewinnung von Prüfkörpern

Formstoffplatten zur Herstellung von Prüfkörpern werden durch Eingießen der Mischung in Metallformen folgender Abmessungen und nachfolgende Aushärtung unter den in Tabelle 2 angegebenen Bedingungen gewonnen:

- Aluminium-Formen zur Herstellung von Platten mit den Abmessungen 135 x 135 x 4 mm. Aus den mit Hilfe dieser Formen hergestellten Formstoff-Platten werden Prüfkörper mit den Abmessungen 60 x 10 x 4 mm zur Bestimmung von Biegefestigkeit und Durchbiegung nach VSM-Norm 77 103, Schlagbiegefestigkeit nach VSM-Norm 77 105 und der Gewichtszunahme nach Wasserlagerung herausgesägt.
- Aluminium-Form zur Herstellung von Prüfkörpern mit den Abmessungen 120 x 10 x 4 mm zur Bestimmung der Formbeständigkeit in der Wärme (Heat Distortion nach Iso R 75). An einer kleinen Probe wird thermoanalytisch die Glasumwandlungstemperatur bestimmt.

b) Herstellung von Prüfkörpern für die Bestimmung der Zugscherfestigkeit

Auf durch Schleifen aufgerauhte und durch Waschen mit Aceton gereinigte Enden von Prüfstreifen aus Anticorodal B mit den Abmessungen 170 x 25 x 1,5 mm wird Harz/Härtermischung aufgebracht. Je 2 dieser Prüfstreifen sind mit Hilfe einer Lehre so justiert, daß sich die mit Harz/Härtermischung bestrichenen Enden 10 mm überlappen. Sie werden dann in dieser Stellung mit einer Schlauchklammer fixiert und unter den in der Tabelle angegebenen Bedingungen ausgehärtet.

c) Herstellung von Prüfkörpern für die lacktechnische Untersuchung

Durch Waschen mit Trichloräthylen entfettete Eisenbleche mit den Abmessungen 350 x 70 x 0,8 mm werden mit Hilfe eines Ziehdreiecks mit einem 50 µ dicken Film aus der jeweiligen Harz/Härtermischung überzogen. Nach der Härtung unter den in der Tabelle 3 angegebenen Bedingungen dienen die lackierten Blechstreifen der Bestimmung von Erichsentiefung, Schlagtiefung und Dornbiegewinkel sowie der chem. Beständigkeit der Lackfilme. Dazu wird jeweils ein Tropfen der jew. Prüfflüssigkeit auf den Film aufgebracht, mit einem Uhrglas überdeckt und nach einer Stunde Einwirkung bei 23° C das Aussehen des Films beurteilt.

Die Zusammensetzungen der Mischungen gemäß den Beispielen III bis VIII, die Werte der Gebrauchsdauer und ein Teil der Prüfungswerte der gehärteten Epoxidharzmischungen sind in Tabelle 2 zusammengestellt. Weitere Prüfwerte für Lacksysteme folgen in Tabelle 3 (Beispiele IX bis XIV).

Die in den Tabellen 2 und 3 zusammengestellten Resultate belegen, daß sich die neuen Amine sehr gut als Härter für Epoxidharze eignen. Bemerkenswert ist, daß trotz der deutlich verlängerten Gebrauchsdauer eine Härtung von Formstoffen und auch dünnen Filmen bei Raumtemperatur möglich bleibt. Voll ausgehärtete Formstoffe und Filme zeichnen sich durch gute mech. Eigenschaften aus. Bemerkenswert ist die hohe Schlagzähigkeit der Formstoffe.

Tabelle 2

| | Beispiel III | | IV | | V | | VI | | VII | | VIII | | Vergleichsbeispiel 1 | |
|---|---|---|---|---|---|---|---|---|---|---|---|---|---|---|
| Härter hergestellt nach Beispiel | 1 bzw. 2 | | 7 | | 5 bzw. 6 | | 14 | | 21 | | 22 | | THMD | |
| Gew.-% Härter, bezogen auf das Epoxidharz | 34,6 | | 30,8 | | 37,3 | | 39,2 | | 42,9 | | 46,7 | | 21,3 | |
| Gebrauchsdauer bei 40°C | 139 Min. | | 114 Min. | | 85 Min. | | 111 Min. | | | | | | 37 Min. | |
| Härtung<br>A 1 Woche 25°<br>B 24 h 40° + 6 h 100° | A | B | A | B | A | B | A | B | A | B | A | B | A | B |
| Schlagbiegefestigkeit ($KJ/m^2$) | 14 | 56 | 12,5 | 73 | 8,3 | 53 | | 40 | | | | | 7,2 | 15,8 |
| Biegefestigkeit*) ($N/mm^2$) | 88/43 | 86/65 | 77 | 30/51 | 76 | 90/58 | | 102/74 | | | | | 88 | 93 |
| Durchbiegung*) (mm) | 5,5/17 | 8,0/18,5 | 3,8 | 8/18 | 3,9 | 7,5/19 | | 8,0/15,5 | | | | | 3,7 | 14,5 |
| Heat Distortion (°C) | 60 | 90 | 63 | 97 | 57 | 85 | | 96 | | | | | 67 | 113 |
| GUT auf TA2000 (°C) | 61 | 95 | 57 | 109 | 57 | 98 | | 108 | 51 | 93 | 50 | 92 | 61 | 118 |
| $H_2O$ Aufn. u. 4d. Rt (%) | 0,18 | 0,26 | 0,23 | 0,22 | 0,25 | 0,22 | | 0,11 | | | | | 0,20 | 0,18 |
| $H_2O$ Aufn. u. 1hKw (%) | 0,47 | 0,33 | 0,47 | 0,30 | 0,45 | 0,29 | | 0,33 | | | | | 0,39 | 0,30 |
| Zugscherfestigkeit ($N/mm^2$) | 8,9 | 14,1 | 8,2 | 15,1 | 7,9 | 13,3 | | 20,2 | | | | | 6,9 | 9,1 |

*) Wenn zwei Werte, 1. bei max. Last, 2. beim Bruch.

Tabelle 3

| | Beispiel | | | | | | |
|---|---|---|---|---|---|---|---|
| | IX | X | XI | XII | XIII | XIV | Vergleichs-beispiel 1 |
| Härter, hergestellt nach Beispiel | 1 bzw. 2 | 7 | 5 bzw. 6 | 14 | 21 | 22 | THMD |
| Gew.-% Härter, bezogen auf das Epoxidharz | 34,6 | 30,8 | 37,3 | 39,2 | 42,9 | 46,7 | 21,3 |
| Härtung | jeweils 1 Woche bei Raumtemperatur | | | | | | |
| Aussehen des Lackfilms | i. o. hoher Glanz | farblos matt rauh | i. o. farblos | i. o. hoher Glanz | i. o. hoher Glanz | i. o. hoher Glanz | schwach klebrige Oberfläche |
| Erichsentiefung (mm) | 6,8 | 6,7 | 8 | 3,4 | 3,6 | 3,6 | 2,3 |
| Impact-Test (cm/kg Hammer) | 30/1 | 40/1 | 30/1 | <10/1 | <10/1 | <10,1 | <10,1 |
| Dornbiege (∢) | 180 | 180 | 30 | 75 | 100 | 90 | 70 |
| Chemische Beständigkeit*) | | | | | | | |
| 5n $H_2SO_4$ | 1 | 1 | 1 | 1 | 1 | 1 | 1 |
| 5n NaOH | 1 | 1 | 1 | 1 | 1 | 1 | 1 |
| $H_2O$ | 1 | 1 | 1 | 1 | 1 | 1 | 1 |
| Aceton | 1 | 3 | 3 | 3 | 2 | 3 | 2 |
| Cl-Benzol | 2 | 3 | 4 | 3 | 3 | 4 | 3 |

*) 1 Tropfen der jeweiligen Chemikalien während 1 Stunde auf dem Film belassen:
1 = kein Angriff
2 = leicht angegriffen
3 = stark angegriffen.

Beispiele XV bis XXII
(und Vergleichsbeispiele 2 und 3)

In Tabelle 4 sind die Rezepturen weiterer härtbarer Mischungen für Lacke zusammengestellt. Die Mischung der jeweiligen Bestandteile erfolgt in bekannter Weise. Während die lösungsmittelfreien Mischungen gemäß Beispiel XV bis XVIII und Vergleichsbeispiel 2 bei Raumtemperatur hergestellt und appliziert werden können, muß gemäß den Beispielen XIX bis XXIII und Vergleichsbeispiel 3 das feste Epoxidharz erst bei ca. 60° C im Lösungsmittel gelöst werden. Nach Abkühlen auf 20° C wird in üblicher Weise der Härter zugesetzt und gemischt bis der Ansatz homogen ist. Anschließend kann dei Lack appliziert werden.

Als Epoxidharze werden in den Versuchen folgende verwendet:

Harz A

Dieses entspricht dem in den Beispielen III bis XIV eingesetzten Typ.

Harz B

Ein bei Raumtemperatur fester, durch Kondensation von Bisphenol A mit Epichlorhydrin in Gegenwart von Alkali hergestellter Polyglycidyläther mit einem Epoxidgehalt von 2,1 Epoxidäquivalenten/kg und einem Erweichungspunkt von ca. 50° C (Kofier).

Als Aminhärter werden neben dem Produkt gemäß Beispiel 1 bzw. 2 die drei folgenden Addukt-Härter verwendet:

Addukthärter M

Voraddukt, hergestellt durch Umsetzung von 1 Mol Harz A mit 4 Mol des gemäß Beispiel 1 hergestellten Härters (Viskosität 25° C: 4500 mPa s, Amingehalt: 5,6 Val/kg).

Addukthärter N

Voraddukt, hergestellt durch Umsetzung von 1 Mol Harz A mit 2,5 Mol des gemäß Beispiel 10 hergestellten Härters in Benzylalkohol. Der Gehalt an Benzylalkohol im Härter beträgt 27% (Viskosität 25° C: 3700 mPa s, Amingehalt 3,6 Val/kg).

Addukthärter O

Voraddukt, hergestellt durch Umsetzung von 1 Mol Harz A mit 3,5 Mol des gemäß Beispiel 1 hergestellten Härters. Als Beschleuniger enthält dieses Produkt 17% Bisphenol A (Viskosität 25° C: 5900 mPa s, Amingehalt 5,2 Val/kg).

Tabelle 4
(Mengenangaben in Gewichtsteilen).

| Beispiel | Harz A | Harz B | Härter gemäß Beispiel 1 bzw. 2 | 2,4,6-Tri-(dimethylamino-methyl)phenol | Addukt-härter M | Addukt-härter N | Addukt-härter O | Xylol | Butanol | Vergleichshärter Trimethylhexa-methylendiamin |
|---|---|---|---|---|---|---|---|---|---|---|
| XV | 100 | | 34 | 2 | | | | | | |
| XVI | 100 | | | | 50 | | | | | |
| XVII | 100 | | | | | 90 | | | | |
| XVIII | 100 | | | | | | 50 | | | |
| XIX | | 100 | 14 | | | | | 40 | 10 | |
| XX | | 100 | 12 | 2 | | | | 40 | 10 | |
| XXI | | 100 | | | 21 | | | 44 | 11 | |
| XXII | | 100 | | | | 38 | | 48 | 12 | |
| XXIII | | 100 | | | | | 21 | 44 | 11 | |
| Vergleichsbeispiel 2 | 100 | | | 2 | | | | | | 20 |
| Vergleichsbeispiel 3 | | 100 | | | | | | 36 | 9 | 8 |

Die Lackmischungen und die gehärteten Systeme werden nach folgenden Vorschriften geprüft:

- Viskosität nach DIN 53 015 bis 25° C.
- Gelierzeit von 100 ml bei 20° C. Messung erfolgt mittels Tecam-Gerät der Fa. Techne (Cambridge) Ltd, Duxford Cambridge Uk.
- Staubtrocken- und Durchärtungszeit, 200 µm Film bei lösungsmittelfreien Anwendungen, 50 µm Filmdicke bei lösungsmittelhaltiger Applikation, gemessen mittels Universal-Trockenzeitprüfgerät (Typ 338) der Fa. Erichsen bei 20° C 65% RF.
- Härte nach Persoz bei 20° C 65% RF. Angabe der Härte erfolgt in Sekunden. Gerät Typ 300 der Firma Erichsen. Filmdicke bei lösungsmittelfreier Applikation 200 µm, bei lösungsmittelhaltiger Anwendung 50 µm.
- Tiefziehfähigkeit nach Erichsen. DIN 53 152, gemessen bei 20° C 65% RF. Lösungsmittelfreie Applikation 200 µm Filmdicke, lösungsmittelhaltige Applikation 50 µm Filmdicke. Angabe erfolgt in mm.
- Impacttest, Schlag erfolgt auf den Film, Prüfung bei 20° C 65% RF. Gewicht des Prüfhammers 1 kg, Durchmesser der aufschlagenden Halbkugel 2 cm. Lösungsmittelfreie Applikation 200 µm Filmdicke, lösungsmittelhaltige Applikation 50 µm Filmdicke, Angabe erfolgt in cm/kg.
- Dornbiegetest, 15 mm Dorn (DIN 53 152) bei 20° C 65% RF Filmdicke lösungsmittelfreie Applikation 200 µm, lösungsmittelhaltig 50 µm.

Die Versuchsergebnisse sind in Tabelle 5 zusammengestellt.

Tabelle 5

| | Beispiel Nr. | | | | | | | | | Vergleichs-beispiel 2 | Vergleichs-beispiel 3 |
|---|---|---|---|---|---|---|---|---|---|---|---|
| | XV | XVI | XVII | XVIII | XIX | XX | XXI | XXII | XXIII | | |
| Viskosität, mPas | 600 | 5400 | 3300 | 6000 | 1500 | 1200 | 1600 | 1000 | 1700 | 700 | 2700 |
| Gelierzeit, h | 11 : 15 | 9 : 10 | 7 : 00 | 1 : 45 | 50 | 18 | 47 | 49 | 41 | 1 : 15 | 3 |
| Staubtrockenzeit, h | 30 | 30 | 26 | 12 | 19 | 12 | 24 | 26 | 22 | 36 | 5 |
| Durchhärtungszeit, h | >30 | >30 | >30 | 20 | >30 | >30 | >30 | >30 | >30 | 6 | 8 |
| Härte nach Persoz nach 1 Wo 20°C, s | 350 | 300 | 200 | 360 | 80 | 110 | 90 | 80 | 70 | 330 | 150 |
| Erichsentest | | | | | | | | | | | |
| 1 Wo 20°C, mm | 3.0 | 6.0 | 9.5 | 5.5 | 9.5 | 9.5 | 9.5 | 9.5 | 9.5 | 1.0 | 7.0 |
| 1 Wo 60°C, mm | 6.0 | 5.0 | 8.5 | 3.0 | 9.0 | 9.5 | 9.0 | 9.5 | 9.5 | 5.0 | 5.5 |
| Impacttest | | | | | | | | | | | |
| 1 Wo 20°C, cmkg | 60 | 20 | 90 | 50 | 90 | 90 | 90 | 90 | 90 | 20 | 70 |
| 1 Wo 60°C, cmkg | 90 | 50 | 90 | 80 | 90 | 90 | 90 | 90 | 90 | 80 | 90 |
| Dornbiegetest | | | | | | | | | | | |
| 1 Wo 20°C, ∢° | 10 | 20 | 180 | 20 | 180 | 180 | 180 | 180 | 180 | 10 | 90 |
| 1 Wo 60°C, ∢° | 180 | 30 | 180 | 70 | 180 | 180 | 180 | 180 | 180 | 90 | 180 |

**Patentansprüche**

1. Eine Verbindung der Formel I

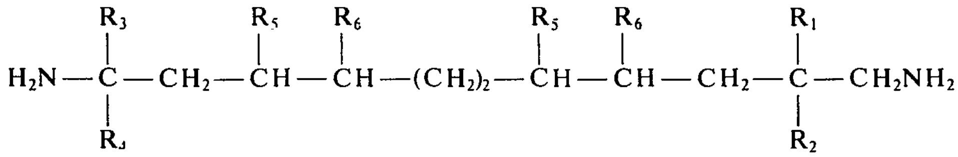

(I)

worin

$R_1$ Alkyl mit 1 – 12 C-Atomen,
$R_2$ Wasserstoff oder Alkyl mit 1 – 12 C-Atomen,
$R_3$ Alkyl mit 1 – 12 C-Atomen, Cycloalkyl mit 4 – 12 Ring-C-Atomen, Aralkyl mit 7 oder 8 C-Atomen, Aryl, das mit einer oder mehreren $C_1 – C_4$-Alkylgruppen substituiert sein kann, Pyridyl, Furyl oder Thienyl,
$R_4$ Wasserstoff, Alkyl mit 1 – 12 C-Atomen, Cycloalkyl mit 4 – 12 Ring-C-Atomen, Aralkyl mit 7 oder 8 C-Atomen oder gegebenenfalls mit einer oder mehreren $C_1 – C_4$-Alkylgruppen substituiertes Aryl oder
$R_1$ und $R_2$ und/oder $R_3$ und $R_4$ zusammen Alkylen mit 3 – 11 C-Atomen und
$R_5$ und $R_6$ unabhängig voneinander Wasserstoff oder Methyl

bedeuten.

2. Eine Verbindung der Formel I nach Anspruch 1, worin $R_1$ Alkyl mit 1 – 5 C-Atomen, $R_2$ Wasserstoff oder Alkyl mit 1 – 5 C-Atomen, $R_3$ Alkyl mit 1 – 7 C-Atomen, Cycloalkyl mit 5 – 8 C-Atomen oder unsubstituiertes Phenyl, $R_4$ Wasserstoff oder Alkyl mit 1 – 5 C-Atomen oder $R_1$ und $R_2$ zusammen Alkylen mit 4 – 7 C-Atomen und $R_5$ und $R_6$ je Wasserstoff bedeuten.

3. Eine Verbindung der Formel I nach Anspruch 1, worin $R_1$ Alkyl mit 1 – 5 C-Atomen, $R_2$ Alkyl mit 1 – 5 C-Atomen oder Wasserstoff oder $R_1$ und $R_2$ zusammen Alkylen mit 4 – 7 C-Atomen, $R_3$ verzweigtes Alkyl mit 3 – 7 C-Atomen oder Cycloalkyl mit 5 – 8 C-Atomen und $R_4$, $R_5$ und $R_6$ je Wasserstoff bedeuten.

4. Eine Verbindung der Formel I nach Anspruch 1, worin $R_1$ und $R_2$ je Methyl oder Äthyl oder zusammen $C_{4-7}$-Alkylen, besonders Pentamethylen, $R_3$ Isopropyl, 3-Pentyl, $C_{5-8}$-Cycloalkyl, besonders Cyclohexyl, und $R_4$, $R_5$ und $R_6$ je Wasserstoff bedeuten.

5. Eine Verbindung nach Anspruch 1 der Formel IX

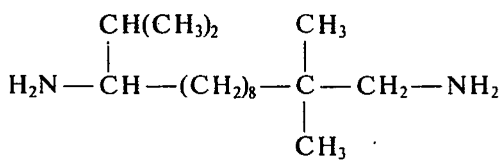

(IX)

6. Eine Verbindung nach Anspruch 1 der Formel X

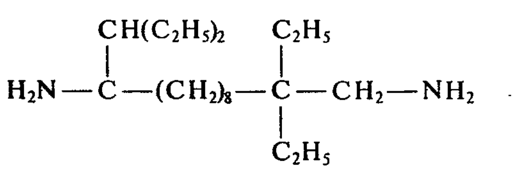

(X)

7. Eine Verbindung nach Anspruch 1 der Formel XI

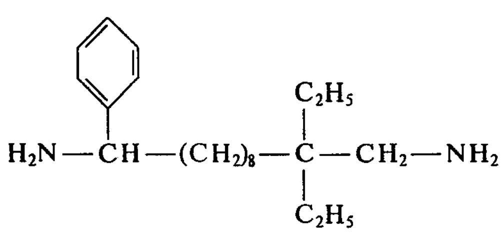

(XI)

8. Ein Verfahren zur Herstellung einer Verbindung der Formel I nach Anspruch I, dadurch gekennzeichnet, daß man

A) eine Verbindung der Formel II

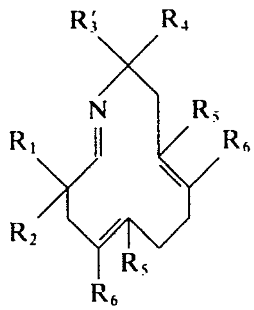

(II)

worin $R_1$, $R_2$ und $R_4$ bis $R_6$ die unter Formel I angegebene Bedeutung haben und $R_3'$ dasselbe wie $R_3$ in Formel I oder, wenn $R_4$ Wasserstoff ist, auch

$$-CH=CH-Alkyl$$

oder

$$-C(Alkyl)=CH-Alkyl$$

mit je 1–4 C-Atomen in den Alkylteilen darstellt,

mit einer Verbindung der Formeln IIIa oder IIIb

$$H_2N-Y \quad \text{(IIIa)}$$

oder

$$[H_2NY \cdot H^+]_n \quad X^{n-} \quad \text{(IIIb)}$$

worin X das Anion einer anorganischen, unter den Reaktionsbedingungen nicht oxidierenden Säure, n eine der Wertigkeit von X entsprechende ganze Zahl und Y eine der Gruppen

$-OH$ $-NH_2$ 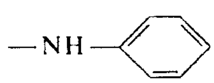

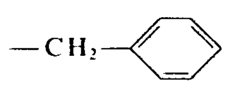 oder $-NHCONH_2$

darstellen, zu einer Verbindung der Formel IVa umsetzt

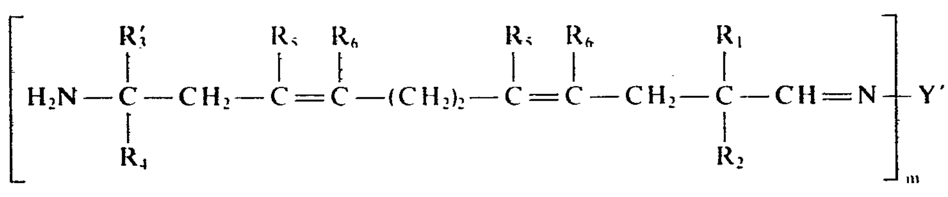

(IVa)

in der m die Zahl 1 oder 2 und Y' bei m = 1 die obige Bedeutung hat und bei m = 2 die direkte Bindung darstellt, oder

B) eine Verbindung der Formel II katalytisch zu einer Verbindung der Formel V

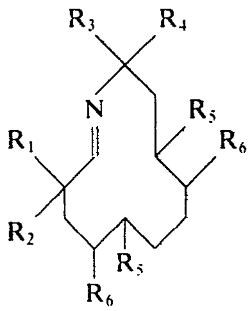

(V)

hydriert, wobei $R_1$ bis $R_6$ die unter Formel I angegebene Bedeutung haben, und die Verbindung der Formel V mit einer der Verbindungen der Formeln III a oder III b zu einer Verbindung der Formel IV b

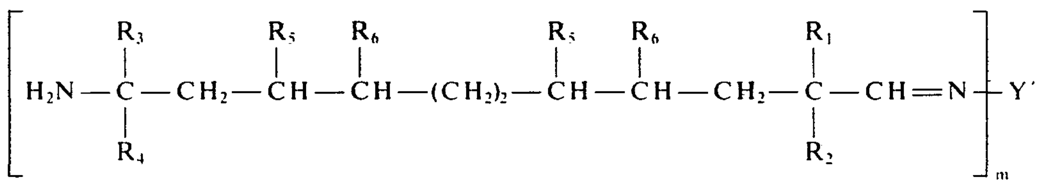

(IV b)

in der m und Y' die in Formel IVa angegebene Bedeutung haben, umsetzt und die Verbindung der Formel IVa oder IVb katalytisch zu einer Verbindung der Formel I hydriert.

9. Ein Verfahren nach Anspruch 8, dadurch gekennzeichnet, daß man eine Verbindung der in einem der Ansprüche 1 – 7 genannten Art herstellt.

10. Härtbare Gemische, enthaltend

a) ein Diamin der Formel I gemäß Anspruch 1,
b) eine Polyepoxidverbindung (X) mit durchschnittlich mehr als einer Epoxidgruppe im Molekül,

wobei in den Gemischen auf 1 Äquivalent Epoxidgruppen der Polyepoxidverbindung (X) 0,5 bis 1,5 Äquivalente an Stickstoff gebundene, aktive Wasserstoffatome des jeweiligen Diamins der Formel I kommen.

11. Gemische nach Anspruch 10, dadurch gekennzeichnet, daß sie das Diamin der Formel I in Form eines Addukthärters (E) mit einer Aminzahl von 3 bis 7 Val/kg aus dem Diamin der Formel I und einer flüssigen Epoxidverbindung (Z) mit durchschnittlich mehr als einer Epoxidgruppe im Molekül und gegebenenfalls Phenylglycid enthalten, wobei in den Gemischen auf 1 Äquivalent Epoxidgruppen der Polyepoxidverbindung (X) 0,8 bis 1,2 Äquivalente an die Stickstoffatome des Addukthärters (E) gebundene, aktive Wasserstoffatome kommen.

## Claims

1. A compound of the formula I

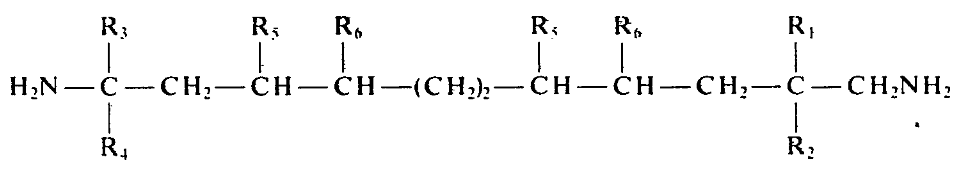

(I)

wherein

$R_1$ is alkyl having 1 – 12 C atoms,
$R_2$ is hydrogen or alkyl having 1 – 12 C atoms,
$R_3$ is alkyl having 1 – 12 C atoms, cycloalkyl having 4 – 12 ring C atoms, aralkyl having 7 or 8 C atoms, aryl which is unsubstituted or substituted by one or more $C_1 - C_4$-alkyl groups, pyridyl, furyl or thienyl,

$R_4$ is hydrogen, alkyl having 1 – 12 C atoms, cycloalkyl having 4 – 12 ring C-atoms, aralkyl having 7 or 8 C atoms, or aryl which is unsubstituted or substituted by one or more $C_1 – C_4$-alkyl groups, or
$R_1$ and $R_2$ and/or $R_3$ and $R_4$ together are alkylene having 3 – 11 C atoms, and
$R_5$ and $R_6$ independently of one another are hydrogen or methyl.

2. A compound of the formula I according to Claim 1 wherein $R_1$ is alkyl having 1 – 5 C atoms, $R_2$ is hydrogen or alkyl having 1 – 5 C atoms, $R_3$ is alkyl having 1 – 7 C atoms, cycloalkyl having 5 – 8 C atoms or unsubstituted phenyl, $R_4$ is hydrogen or alkyl having 1 – 5 C atoms, or $R_1$ and $R_2$ together are alkylene having 4 – 7 C atoms, and $R_5$ and $R_6$ are each hydrogen.

3. A compound of the formula I according to Claim 1, wherein $R_1$ is alkyl having 1 – 5 C atoms, $R_2$ is alkyl having 1 – 5 C atoms or hydrogen, or $R_1$ and $R_2$ together are alkylene having 4 – 7 C atoms, $R_3$ is branched-chain alkyl having 3 – 7 C atoms or cycloalkyl having 5 – 8 C atoms, and $R_4$, $R_5$ and $R_6$ are each hydrogen.

4. A compound of the formula I according to Claim 1, wherein $R_1$ and $R_2$ are each methyl or ethyl, or together are $C_{4-7}$-alkylene, particularly pentamethylene, $R_3$ is isopropyl, 3-pentyl, $C_{5-8}$-cycloalkyl, especially cyclohexyl, and $R_4$, $R_5$ and $R_6$ are each hydrogen.

5. A compound according to Claim 1 of the formula IX

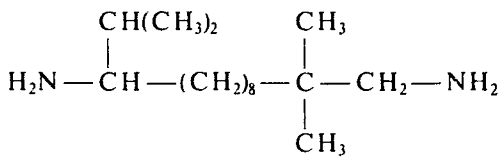

(IX)

6. A compound according to claim 1 of the formula X

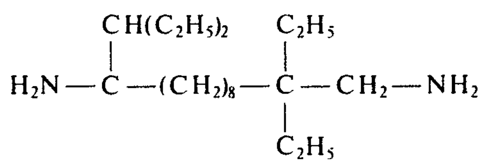

(X)

7. A compound according to Claim 1 of the formula XI

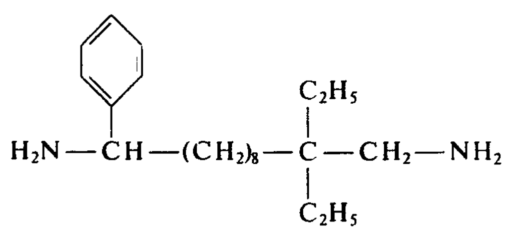

(XI)

8. A process for producing a compound of the formula I according to Claim 1, which process comprises either

A) reacting a compound of the formula II

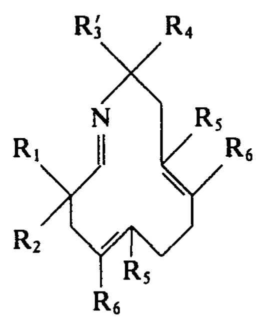

(II)

wherein $R_1$, $R_2$ and $R_4$ to $R_6$ have the meanings given under the formula I, and $R_3'$ is the same as $R_3$ in the formula I or when $R_4$ is hydrogen $R_3$ is also

—CH═CH—alkyl

or

-C(alkyl)═CH—alkyl

having 1–4 C atoms in each of the alkyl moieties,

with a compound of the formula III a or III b

$H_2N—Y$ (III a)

or

$[H_2NY \cdot H^+]_n \quad X^{\ominus n}$ (III b)

wherein X is the anion of an inorganic acid not oxidising under the reaction conditions, n is an integer corresponding to the valency of X, and Y is one of the groups

—OH —$NH_2$ 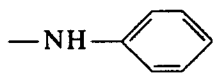

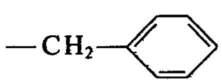 or —$NHCONH_2$

to obtain a compound of the formula IV a

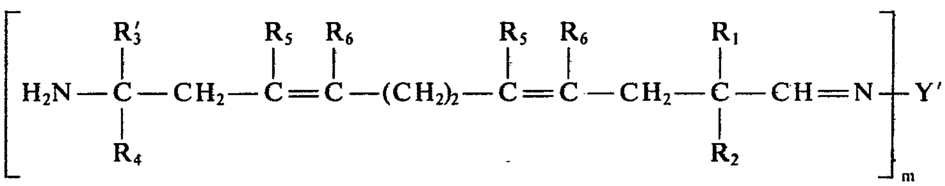 (IV a)

wherein m is the number 1 or 2, and Y′ where m is 1 has the above meaning, and where m is 2 it is the direct bond; or

B) catalytically hydrogenating a compound of the formula II to a compound of the formula V

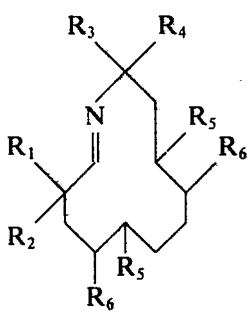 (V)

wherein $R_1$ to $R_6$ have the meanings given under the formula I, and reacting the compound of the formula V with one of the compounds of the formula III a or III b to give a compound of the formula IV b

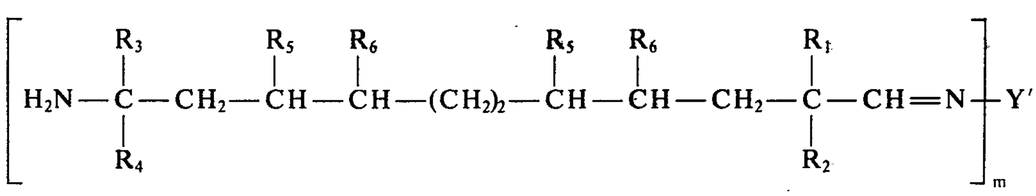

(IV b)

in which m and Y' have the meanings given under the formula IVa, and catalytically hydrogenating the compound of the formula IVa or IVb to obtain a compound of the formula I.

9. A process according to Claim 8, wherein a compound of the type mentioned in any one of Claims 1–7 inclusive is produced.

10. A curable mixture containing

a) a diamine of the formula I according to Claim 1, and
b) a polyepoxide compound (X) having on average more than one epoxide group in the molecule,

there being in the mixture, to 1 equivalent of epoxide groups of the polyepoxide compound (X), 0.5 to 1.5 equivalents of active hydrogen atoms, which are bound to nitrogen, of the respective diamine of the formula I.

11. A mixture according to Claim 10, which contains the diamine of the formula I in the form of an adduct curing agent (E) which has an amine number of 3 to 7 gram equivalents per kg and which is formed from the diamine of the formula I and a liquid epoxide compound (Z) having on average more than one epoxide group in the molecule, and optionally phenylglycide, there being in the mixture, to 1 equivalent of epoxide groups of the polyepoxide compound (X), 0.8 to 1.2 equivalents of active hydrogen atoms bound to the nitrogen atoms of the adduct curing agent (E).

**Revendications**

1. Composé répondant à la formule I

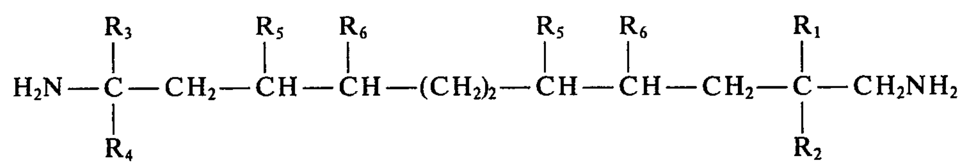

(I)

dans laquelle

$R_1$ représente un radical alkyle contenant de 1 à 12 atomes de carbone,
$R_2$ représente l'hydrogène ou un radical alkyle contenant de 1 à 12 atomes de carbone,
$R_3$ représente un radical alkyle contenant de 1 à 12 atomes de carbone, un radical cycloalkyle contenant de 4 à 12 atomes de carbone dans le cycle, un radical aralkyle contenant 7 ou 8 atomes de carbone, un radical aryle éventuellement porteur d'un ou plusieurs radicaux alkyles en $C_1-C_4$, un radical pyridyle, furyle ou thiényle,
$R_4$ représente l'hydrogène, un radical alkyle en $C_1-C_{12}$, un radical cycloalkyle contenant de 4 à 12 atomes de carbone dans le cycle, un radical aralkyle contenant 7 ou 8 atomes de carbone ou un radical aryle éventuellement porteur d'un ou plusieurs radicaux alkyles en $C_1-C_4$, ou
les couples ($R_1$, $R_2$) et/ou ($R_3$, $R_4$) forment un radical alkylène contenant de 3 à 11 atomes de carbone et $R_5$ et $R_6$ représentent chacun, indépendamment l'un de l'autre, l'hydrogène ou un radical méthyle.

2. Composé de formule I selon la revendication 1 dans lequel $R_1$ représente un alkyle en $C_1-C_5$, $R_2$ l'hydrogène ou un alkyle en $C_1-C_5$, $R_3$ un alkyle en $C_1-C_7$, un cycloalkyle en $C_5-C_8$ ou un phényle non substitué, $R_4$ l'hydrogène ou un alkyle en $C_1-C_5$, ou encore $R_1$ et $R_2$ forment ensemble un alkylène en $C_4-C_7$, et $R_5$ et $R_6$ représentent chacun l'hydrogène.

3. Composé de formule I selon la revendication 1 dans lequel $R_1$ représente un alkyle en $C_1-C_5$, $R_2$ und alkyle en $C_1-C_5$ ou l'hydrogène, ou $R_1$ et $R_2$ forment ensemble un alkylène en $C_4-C_7$, $R_3$ représente un alkyle ramifié en $C_3-C_7$ ou un cycloalkyle en $C_5-C_8$, et $R_4$, $R_5$ et $R_6$ représentent chacun l'hydrogène.

4. Composé de formule I selon la revendication 1 dans lequel $R_1$ et $R_2$ représentent chacun un méthyle ou un éthyle ou forment ensemble un alkylène en $C_4-C_7$, en particulier un pentaméthylène, $R_3$ représente un isopropyle, un éthyl-1 propyle ou un cycloalkyle en $C_5-C_8$, en particulier un cyclohexyle, et $R_4$, $R_5$ et $R_6$ représentent chacun l'hydrogène.

5. Composé selon la revendication 1 qui répond à la formule IX

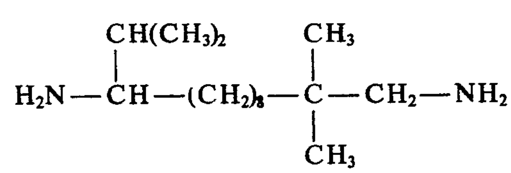

(IX)

6. Composé selon la revendication 1 qui répond à la formule X

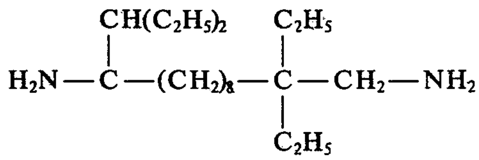

(X)

7. Composé selon la revendication 1 qui répond à la formule XI

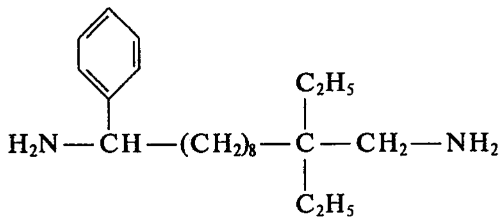

(XI)

8. Procédé de préparation d'un composé de formule I selon la revendication 1, procédé caractérisé en ce que:

A) on fait réagir un composé répondant à la formule II

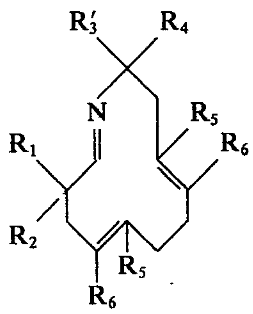

(II)

dans laquelle $R_1$, $R_2$ et $R_4$ à $R_6$ ont les significations données à propos de la formule I, et $R_3'$ a la même signification que $R_3$ dans la formule I ou, lorsque $R_4$ est l'hydrogène, peut également représenter un radical

$$-CH{=}CH-\text{alkyl}$$

ou

$$-C(\text{alkyl}){=}CH-\text{alkyl}$$

contenant de 1 à 4 atomes de carbone dans chacune des parties alkyles, avec un composé répondant à l'une des formules III a et III b

$$H_2N-Y \qquad \text{(III a)}$$

ou

$$[H_2NY \cdot H^+]_n \qquad X^{\ominus n} \qquad \text{(III b)}$$

dans lesquelles X représente l'anion d'un acide minéral non oxydant dans les conditions de la réaction, n est un nombre entier égal à la valence de X, et Y représente l'un des groupes

$-OH$ $-NH_2$ 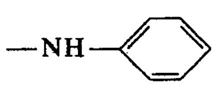

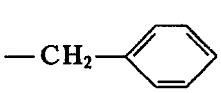 et $-NHCONH_2$

de manière à obtenir un composé répondant à la formule IV a

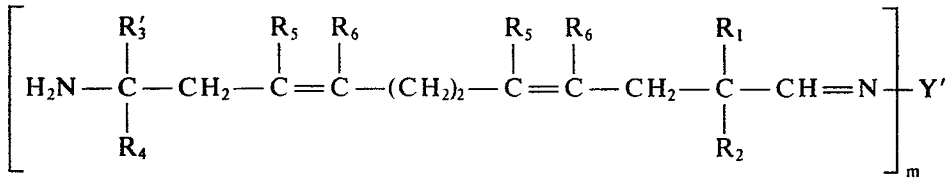

(IV a)

dans laquelle m est égal à 1 ou à 2 et Y', lorsque m est égal à 1, a la signification indiquée ci-dessus et, lorsque m est égal à 2, représente la liaison directe, ou

B) on hydrogène catalytiquement un composé de formule II pour le convertir en un composé répondant à la formule V

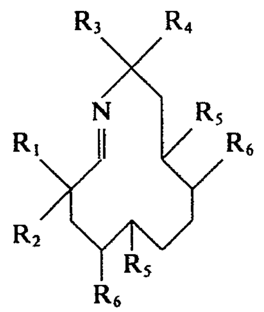

(V)

dans laquelle $R_1$ à $R_6$ ont les significations données à propos de la formule I, et on fait réagir le composé de formule V avec l'un des composés de formule III a ou de formule III b de manière à obtenir un composé répondant à la formule IV b

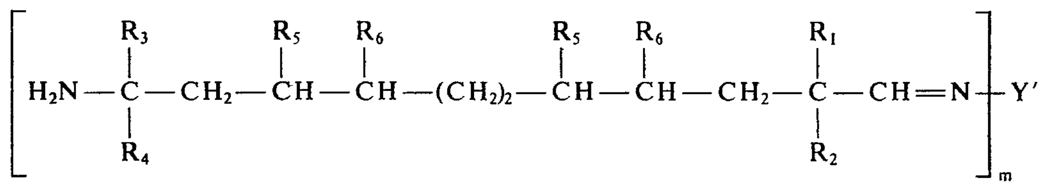

(IV b)

dans laquelle m et Y' ont les significations indiquées à propos de la formule IVa, et on hydrogène catalytiquement le composé de formule IVa ou IVb pour le convertir en un composé de formule I.

9. Procédé selon la revendication 8 caractérisé en ce qu'on prépare un composé du type indiqué dans l'une des revendications 1 à 7.

10. Mélanges durcissables qui contiennent:

a) une diamine de formule I selon la revendication 1 et
b) un composé polyépoxydique (X) contenant en moyenne plus d'un groupe époxy dans la molécule,

dans lesquels il y a, pour 1 équivalent de groupes époxy du composé polyépoxydique (X), de 0,5 à 1,5 équivalent d'atomes d'hydrogène actifs portés par l'azote de la diamine de formule I.

11. Mélanges selon la revendication 10, caractérisés en ce qu'ils contiennent la diamine de formule I sous la forme d'un durcisseur adduct (E) ayant un indice d'amine de 3 à 7 val/kg qui dérive de la diamine de formule I et d'un composé époxydique liquide (Z) renfermant en moyenne plus d'un groupe époxy dans la molécule et éventuellement du phényl-glycide, et dans lesquels il y a, pour 1 équivalent de groupes époxy du composé polyépoxydique (X), de 0,8 à 1,2 équivalent d'atomes d'hydrogène actifs portés par les atomes d'azote du durcisseur (E).